# EUROPEAN PATENT APPLICATION

(11) **EP 4 316 493 A1**
(43) Date of publication of application: **07.02.2024**
(21) Application number: 22779165.4
(22) Date of filing: 02.04.2022
(51) Int. Cl.: A61K 31/5517, A61K 31/475, A61K 31/44, A61P 39/00

(54) **NOVEL USE OF CYCLIC KETONE COMPOUND**

(30) Priority: 02.04.2021 CN 202110362273
(71) Applicant: West China Hospital, Sichuan University, Chengdu, Sichuan 610041 (CN)
(72) Inventor: KE, Bowen, Chengdu, Sichuan 610041 (CN); LIU, Jin, Chengdu, Sichuan 610041 (CN)
(74) Representative: Altmann Stößel Dick Patentanwälte PartG mbB
(86) International application number: PCT/CN2022/084968
(87) International publication number: WO 2022/206985

(57) **Abstract**

Provided is a novel use of a cyclic ketone compound, relating to the pharmaceutical field. A novel pharmaceutical use of the cyclic ketone compound in the field of perioperative medicine is discovered for the first time, and the application prospect is wide.

## Description

### Technical field

The present invention belongs to the field of pharmaceuticals, and specifically relates to a novel use of cyclic ketone compounds in the field of perioperative medicine.

### Background technology

General anesthetics are a type of medicaments that can inhibit the function of the central nervous system, and reversibly cause loss of consciousness, sensation, and reflexes, as well as relaxation of skeletal muscles. They are mainly used for surgical anesthesia. The postoperative recovery time of anesthesia has always been a clinical concern, and is closely related to various factors. For patients, prolonged postoperative anesthesia recovery time will increase the tracheal intubation time, the exposure to general anesthesia risks, the incidence of cardiovascular and pulmonary complications, and affect the prognosis of surgical patients. In addition, the extension of anesthesia recovery time will greatly reduce the utilization efficiency of the operating room, increase the vacancy rate of the operating room, and raise the cost of patients and medical staff. Moreover, with the update of the concept of daytime surgery, rapid anesthesia awakening and good awakening quality are the foundation of promoting daytime surgery and ensuring its safety.

With the increasing requirements for surgical safety and controllability, some antagonists of anesthetic drugs have emerged, such as an opioid antagonist, naloxone; a broad-spectrum muscle relaxant antagonist, neostigmine; and a muscle relaxant specific antagonist of rocuronium, sugammadex. These antagonists can quickly reverse the anesthetic effect of opioids or muscle relaxants, which is beneficial for patients to recover and reverse the anesthetic effect in emergency situations. However, currently, there is a lack of means and methods to address the delayed awakening during prolonged infusion and to reverse the effects of general anesthesia drugs in emergency situations.

At present, the drug with clear antagonistic effects used in clinical practice is flumazenil, which is a specific antagonist of benzodiazepines, and can reverse the anesthetic effects induced by benzodiazepines. However, it has been shown that flumazenil has no significant antagonistic effect on propofol-induced anesthesia. Considering that benzodiazepines are no longer commonly used in clinical anesthesia, there is an urgent need to develop broader spectrum antagonists against general anesthesia, especially those against common general anesthetics such as propofol and etomidate.

### Summary of the invention

The object of the present invention is to provide a novel use of cyclic ketone compounds in the field of perioperative medicine.

The present invention provides the use of compounds represented by formula I, or stereoisomers thereof, or deuterated derivatives thereof, or tritiated derivatives thereof, or metabolites thereof, or prodrugs thereof, or salts thereof, or solvates thereof in the manufacture of anesthetic and/or sedative antagonists: 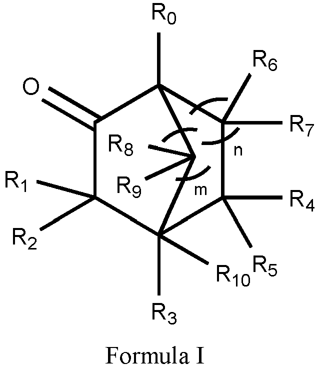
wherein, n is 0 or 1;
m is 0 or 1; when m is 1, R₁₀ is absent;
R₀ is selected from the group consisting of L₀, L₁, L₂, and Rₓ₁;
L₀, L₁, and L₂ are each independently selected from absence, and substituted or unsubstituted following groups: NHCO, NHCS, CO, COO, OCO, OCOO, C(SO), O, S, SO, SO₂, NH, CH=N, CONH, NH(CS)S(CH₂), C₁₋₃ alkylene, C₂₋₄ alkenylene, and C₂₋₄ alkynylene;
Rₓ₁ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₁ and R₂ are each independently selected from the group consisting of L₃, L₄, L₅, Rₓ₂, =NL₅Rₓ₂, and =C(L₆Rₓ₃)(L₇Rₓ₄); or R₁ and R₂ are linked to form a ring;
L₃, L₄, L₅, L₆, and L₇ are each independently selected from absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₂, Rₓ₃, and Rₓ₄ are each independently selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₃ and R₁₀ are each independently selected from the group consisting of L₈, L₉, L₁₀, and Rₓ₅;
L₈, L₉, and L₁₀ are each independently selected from the group consisting of absence, as well as substituted or unsubstituted following groups: NHCO, NHCS, CO, COO, OCO, OCOO, C(SO), O, S, SO, SO₂, NH, CH=N, CONH, NH(CS)S(CH₂), and C₁₋₃ alkylene;
Rₓ₅ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₄, R₅, R₆, and R₇ are each independently selected from the group consisting of L₁₁, L₁₂, L₁₃, Rₓ₆, =NL₁₃Rₓ₆, =C(L₁₄Rₓ₇)( L₁₅Rₓ₈); L₁₁, L₁₂, L₁₃, L₁₄, and L₁₅ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₆, Rₓ₇, and Rₓ₈ are each independently selected from the group consisting of hydrogen, deuterium, tritium, halogen, aldehyde group, CN, N₃, NO₂, OAc, as well as substituted or unsubstituted following groups: OSiH₃, OOSiH₃, SO₃H, Sift, CS₂H, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
or, R₁₀ and R₅ are linked to form a ring;
R₈ and R₉ are each independently selected from L₁₆, L₁₇, L₁₈, and Rₓ₉;
L₁₆, L₁₇, and L₁₈ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₉ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl.

Further, said compound is those represented by formula II: 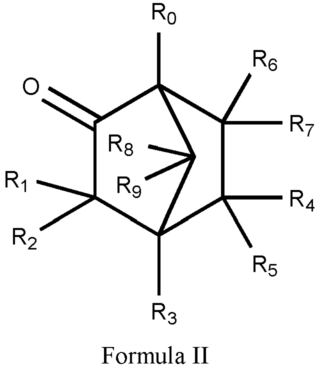
wherein, R₀ is selected from L₀, L₁, L₂, and Rₓ₁;
L₀, L₁, and L₂ are each independently selected from the group consisting of absence, as well as substituted or unsubstituted following groups: NHCO, NHCS, CO, COO, OCO, OCOO, C(SO), O, S, SO, SO₂, NH, CH=N, CONH, NH(CS)S(CH₂), C₁₋₃ alkylene, C₂₋₄ alkenylene, and C₂₋₄ alkynylene;
Rₓ₁ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₁ and R₂ are each independently selected from the group consisting of L₃, L₄, L₅, Rₓ₂, =NL₅Rₓ₂, and =C(L₆Rₓ₃)(L₇Rₓ₄); or R₁ and R₂ are linked to form a ring;
L₃, L₄, L₅, L₆, and L₇ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₂, Rₓ₃, and Rₓ₄ are each independently selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₃ is selected from L₈, L₉, L₁₀, and Rₓ₅;
L₈, L₉, and L₁₀ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₅ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₄, R₅, R₆, R₇ are each independently selected from the group consisting of L₁₁, L₁₂, L₁₃, Rₓ₆, =NL₁₃Rₓ₆, and =C(L₁₄Rₓ₇)( L₁₅Rₓ₈); L₁₁, L₁₂, L₁₃, L₁₄, and L₁₅ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₆, Rₓ₇, Rₓ₈ are each independently selected from the group consisting of hydrogen, deuterium, tritium, halogen, aldehyde group, CN, N₃, NOz, OAc, as well as substituted or unsubstituted following groups: OSiH₃, OOSiH₃, SO₃H, SiH₃, CS₂H, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₈ and R₉ are each independently selected from the group consisting of L₁₆, L₁₇, L₁₈, and Rₓ₉;
L₁₆, L₁₇, and L₁₈ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₉ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl.

Further, said compound is those represented by formula III: 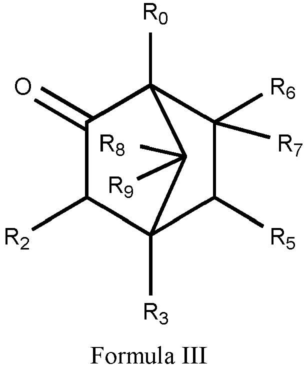
wherein, R₀ is selected from the group consisting of hydrogen, deuterium, tritium, C₁₋₅ alkyl, C₁₋₅ alkyl substituted with 1-3 Rₐ, C₁₋₅ alkoxy, C₁₋₅ alkoxy substituted with 1-3 Rₐ, C₂₋₆ alkenyl, C₂₋₆ alkynyl, saturated or unsaturated heterocyclyl, saturated or unsaturated cycloalkyl, fused cycloalkyl, and fused heterocyclyl; Rₐ is each independently selected from the group consisting of deuterium, tritium, halogen, and hydroxyl;
R₂ is selected from the group consisting of hydrogen, deuterium, tritium, halogen, COORₑ, SRₑ, ORₑ, C₁₋₅ alkyl, =NRₕ₁, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl; Rₑ is selected from the group consisting of hydrogen, deuterium, tritium, and C₁₋₅ alkyl; Rₕ₁ is selected from the group consisting of hydroxyl and C₁₋₅ alkyl;
R₃ is selected from the group consisting of hydrogen, deuterium, tritium, hydroxyl, halogen, C₁₋₅ alkyl, L_{f}COOR_{f}, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl; L_{f} is absence or C₁₋₃ alkylene; Rf is selected from the group consisting of hydrogen, deuterium, tritium, and C₁₋₅ alkyl;
R₅ is selected from the group consisting of hydrogen, deuterium, tritium, halogen, hydroxyl, OCOR_{b}, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl; R_{b} is C₁₋₅ alkyl;
R₆ and R₇ are each independently selected from the group consisting of hydrogen, deuterium, tritium, halogen, C₁₋₅ alkyl, COOR_{g}, C₁₋₅ alkoxy, C_{2~6} alkenyl, C_{2~6} alkynyl, saturated or unsaturated heterocyclyl, saturated or unsaturated cycloalkyl, fused cycloalkyl, and fused heterocyclyl; R_{g} is selected from the group consisting of hydrogen, deuterium, tritium, and C₁₋₅ alkyl;
R₈ and R₉ are each independently selected from the group consisting of hydrogen, deuterium, tritium, C₁₋₅ alkyl, C₁₋₆ alkyl substituted with 1-3 Rₐ, C₁₋₅ alkoxy, C₁₋₅ alkoxy substituted with 1-3 Rₐ, C₂₋₆ alkenyl, C₂₋₆ alkynyl, saturated or unsaturated heterocyclyl, saturated or unsaturated cycloalkyl, fused cycloalkyl, and fused heterocyclyl; R_{c} is each independently selected from the group consisting of halogen, COOR_{d}, hydroxyl, C₁₋₅ alkyl, deuterium, and tritium; R_{d} is selected from the group consisting of hydrogen, deuterium, tritium, and C₁₋₅ alkyl.

Further, R₀ is selected from the group consisting of hydrogen, deuterium, C₁₋₃ alkyl, and C₁₋₃ alkyl substituted with 1-3 Rₐ; Rₐ is each independently selected from the group consisting of deuterium, halogen, and hydroxyl;
R₂ is selected from the group consisting of hydrogen, deuterium, halogen, COORₑ, SRₑ, ORₑ, C₁₋₃ alkyl, and =NRₕ₁; Rₑ is selected from the group consisting of hydrogen, deuterium, and C₁₋₃ alkyl; Rₕ₁ is selected from the group consisting of hydroxyl and C₁₋₃ alkyl;
R₃ is selected from the group consisting of hydrogen, deuterium, hydroxyl, halogen, C₁₋₃ alkyl, and L_{f}COOR_{f}; L_{f} is absence or C₁₋₃ alkylene; R_{f} is selected from the group consisting of hydrogen, deuterium, and C₁₋₃ alkyl;
R₅ is selected from the group consisting of hydrogen, deuterium, halogen, hydroxyl, OCOR_{b}; R_{b} is C₁₋₃ alkyl;
R₆ and R₇ are each independently selected from the group consisting of hydrogen, deuterium, halogen, C₁₋₃ alkyl, and COOR_{g}; R_{g} is selected from the group consisting of hydrogen, deuterium, and C₁₋₃ alkyl;
R₈ and R₉ are each independently selected from the group consisting of hydrogen, deuterium, C₁₋₃ alkyl, and C₁₋₃ alkyl substituted with 1-3 Rₐ; R_{c} is each independently selected from the group consisting of halogen, COOR_{d}, hydroxyl, C₁₋₃ alkyl, and deuterium; R_{d} is selected from the group consisting of hydrogen, deuterium, and C₁₋₃ alkyl.

Further, said compound is those represented by formula IV: 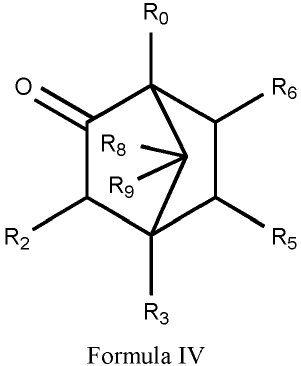
R₀ is selected from the group consisting of methyl, and methyl substituted with 1-3 Rₐ; Rₐ is each independently selected from the group consisting of deuterium, halogen, and hydroxyl;
R₂ is selected from the group consisting of hydrogen, halogen, COOH, SCH₃, methyl, and =N-OH;
R₃ is selected from the group consisting of hydrogen, hydroxyl, halogen, C₁₋₃ alkyl, and L_{f}COOH; L_{f} is methylene;
R₅ is selected from the group consisting of hydrogen, halogen, hydroxyl, OCOR_{b}; R_{b} is methyl;
R₆ is selected from the group consisting of hydrogen, halogen, methyl, and COOH;
R₈ and R₉ are each independently selected from the group consisting of hydrogen, methyl, and methyl substituted with 1-3 Rₐ; R_{c} is each independently selected from the group consisting of halogen, COOH, hydroxyl, methyl, and deuterium.

Further, said compound is selected from the group consisting of

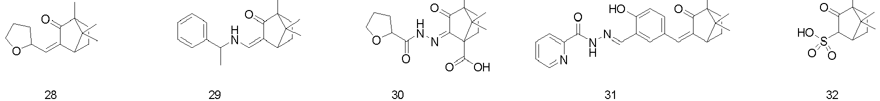

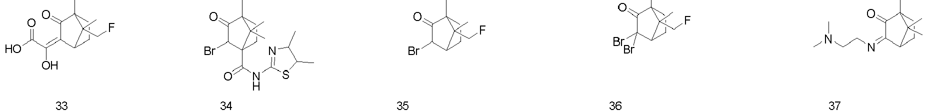

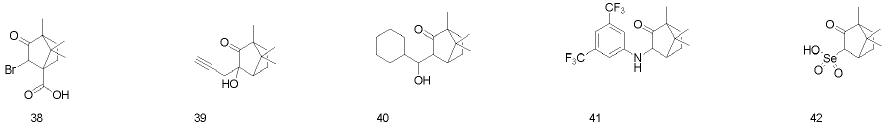

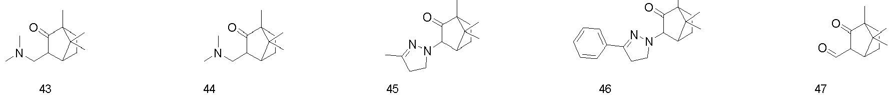

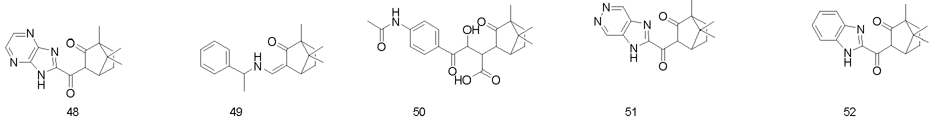

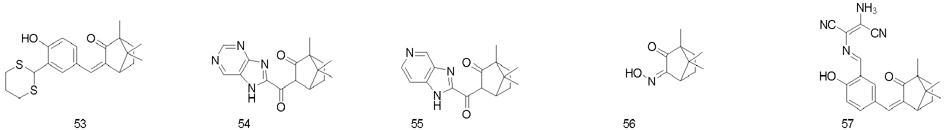

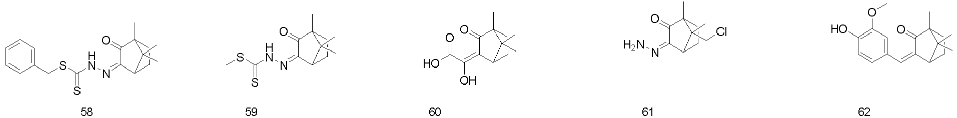

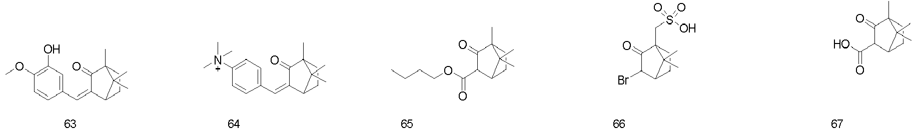

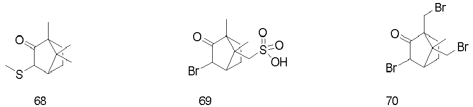

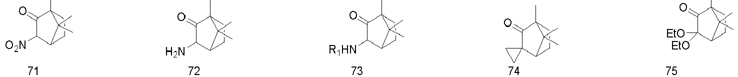

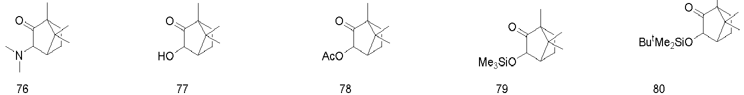

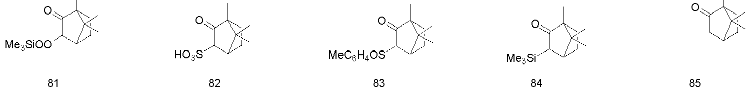

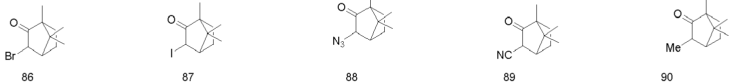

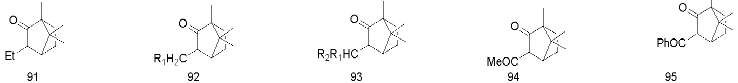

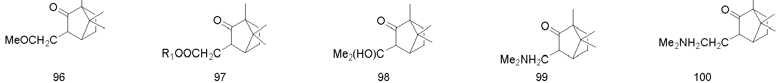

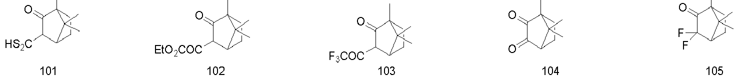

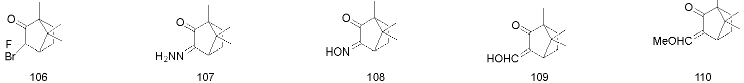

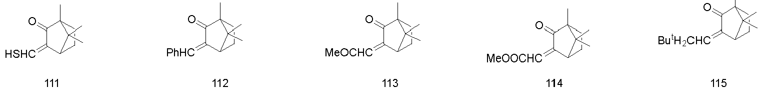

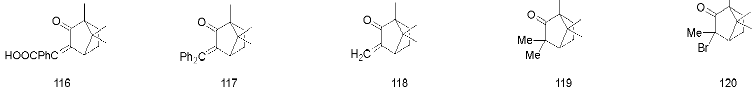

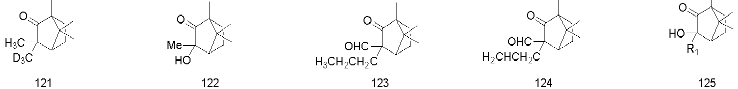

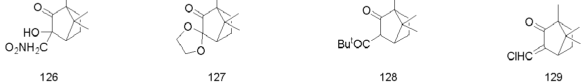

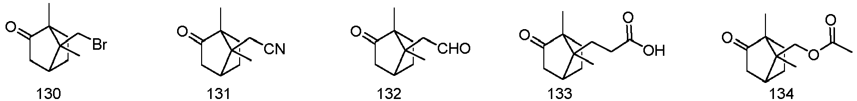

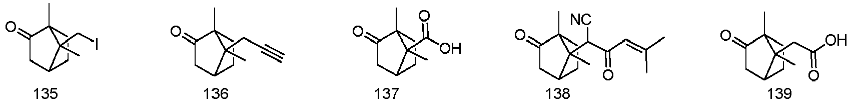

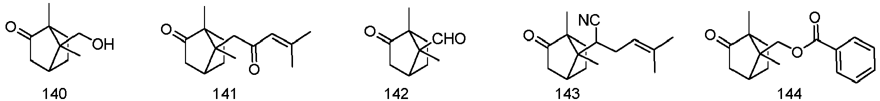

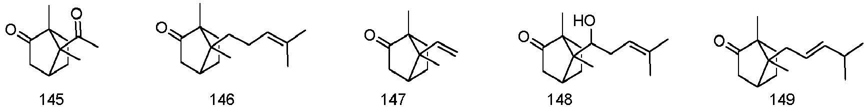

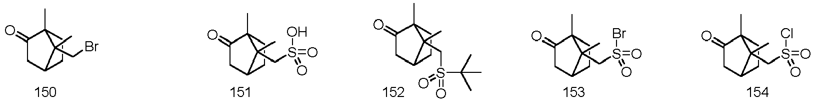

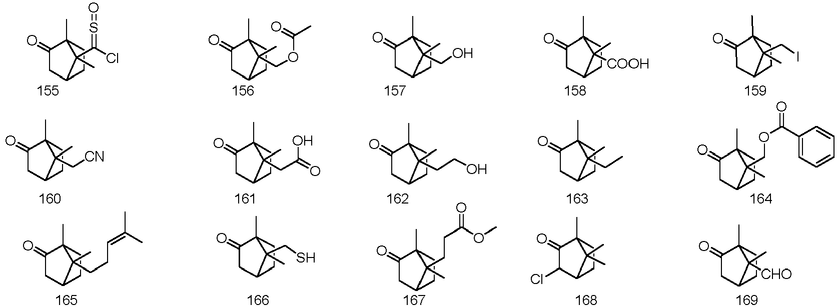

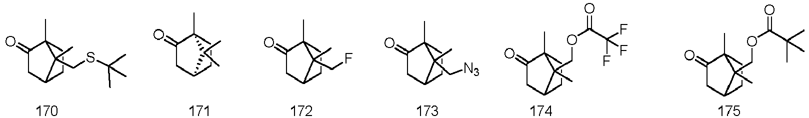

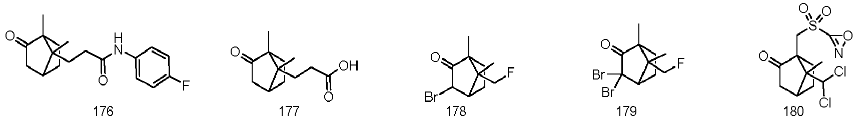

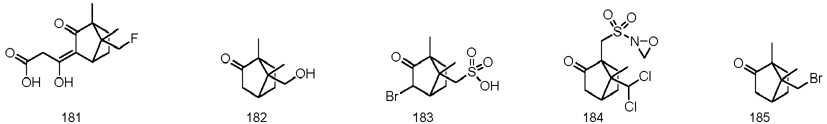

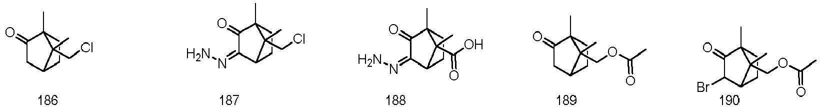

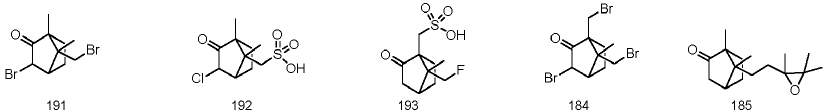

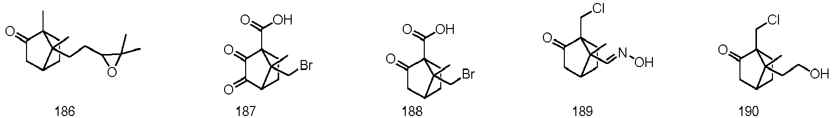

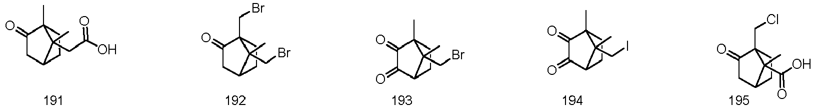

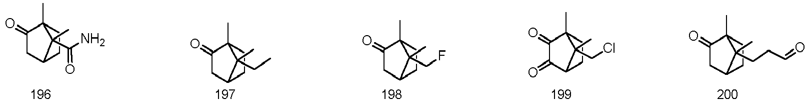

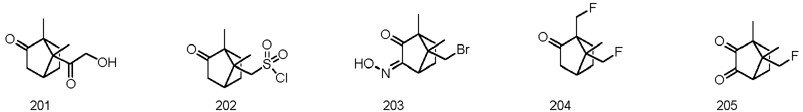

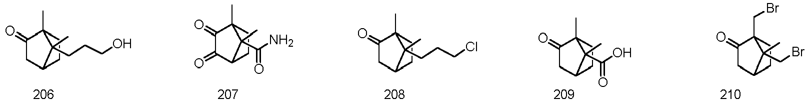

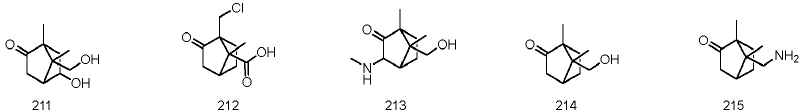

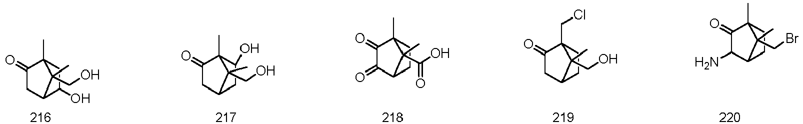

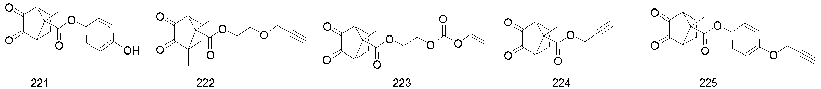

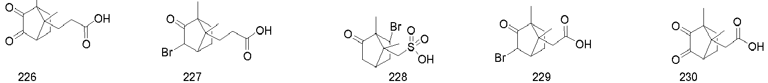

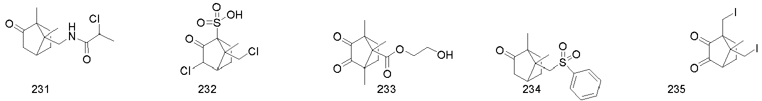

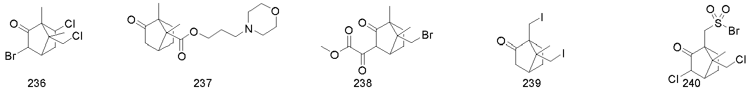

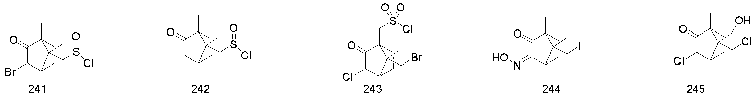

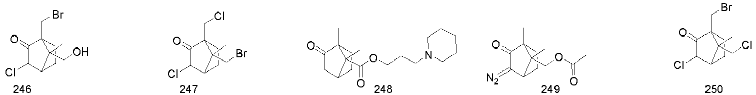

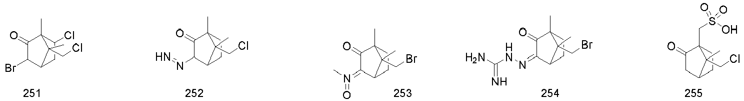

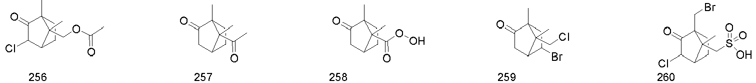

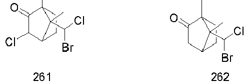

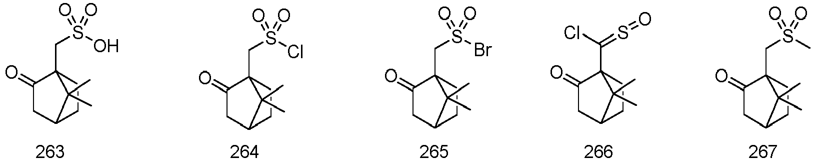

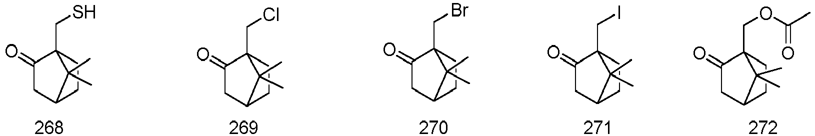

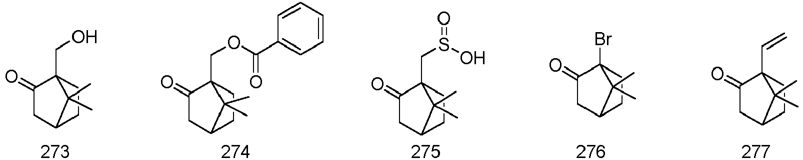

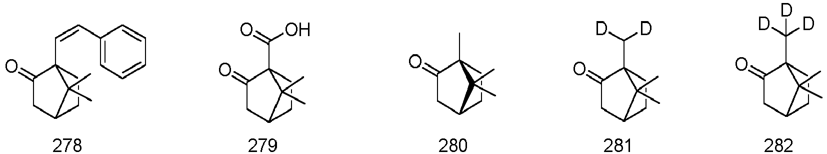

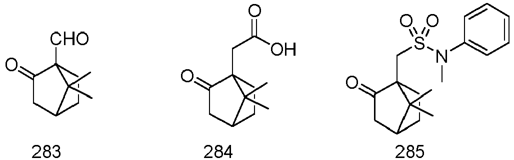

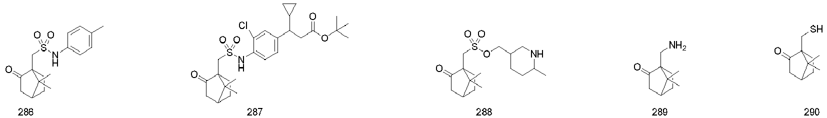

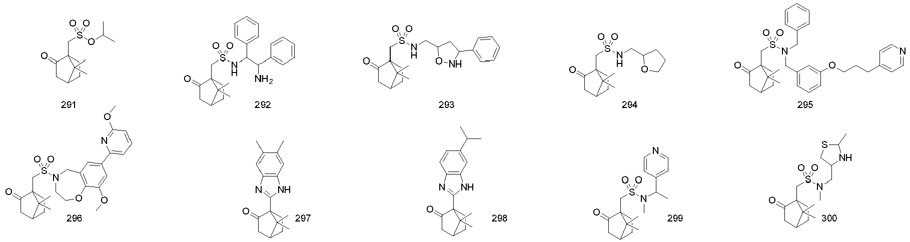

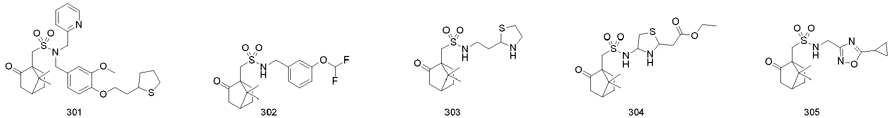

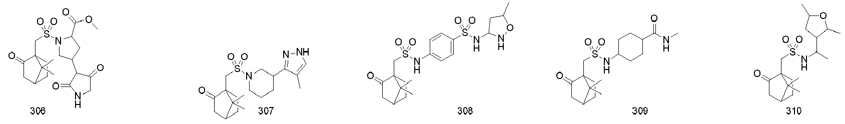

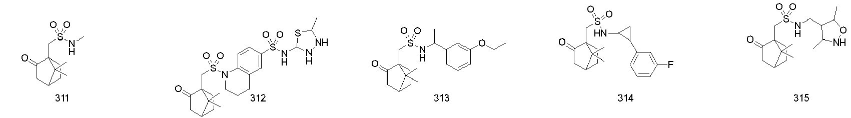

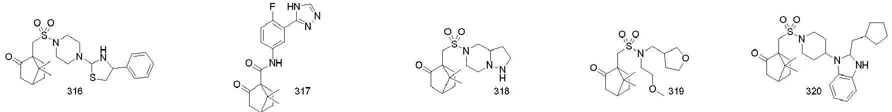

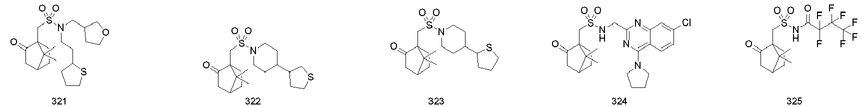

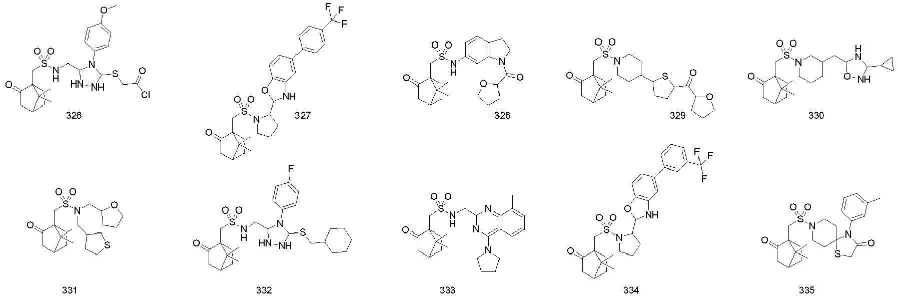

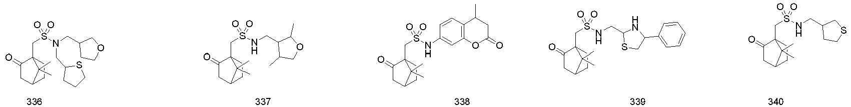

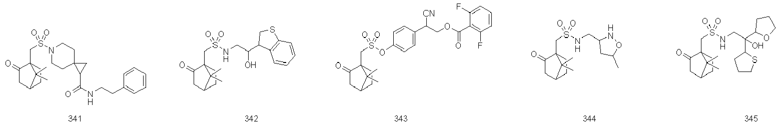

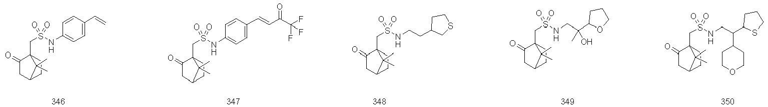

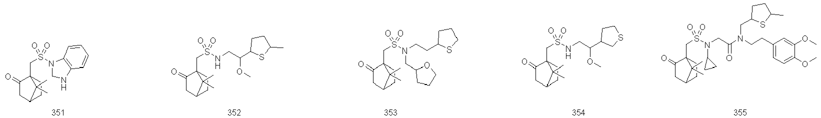

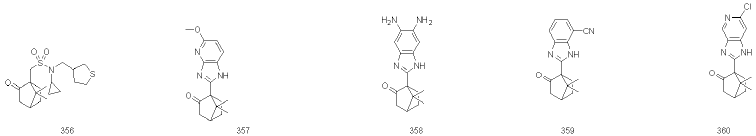

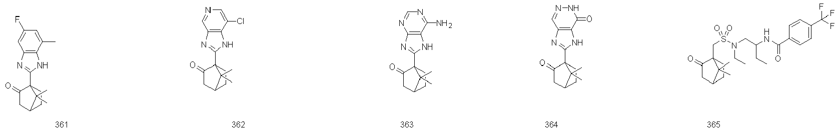

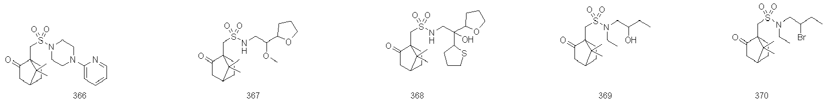

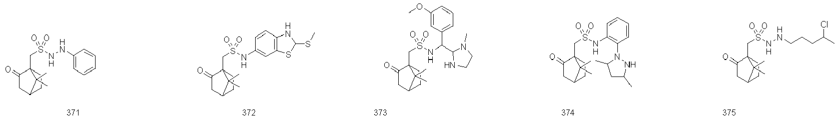

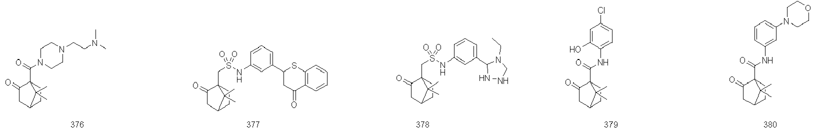

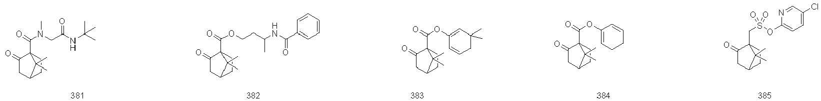

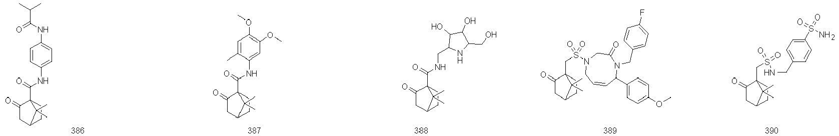

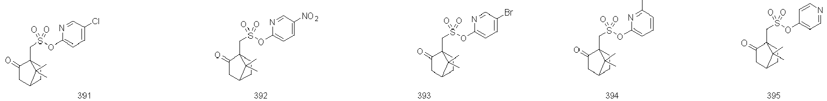

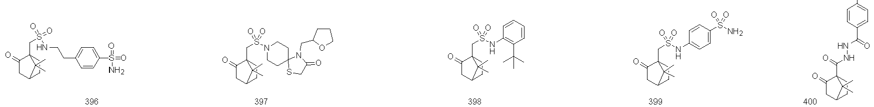

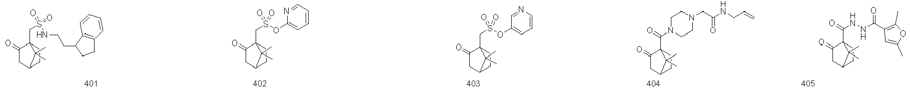

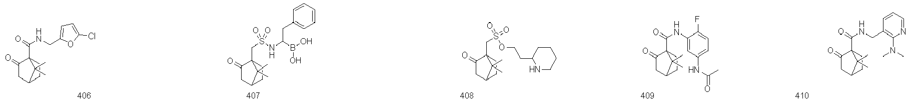

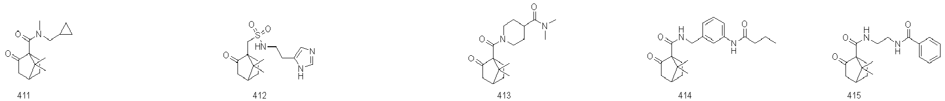

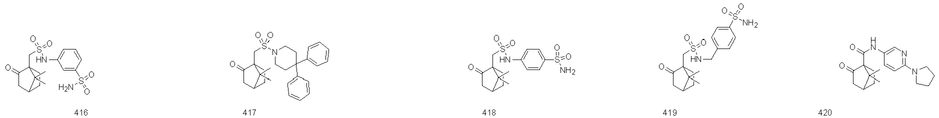

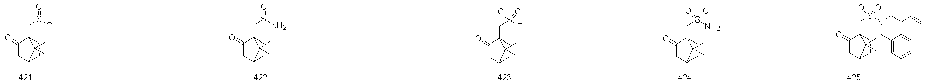

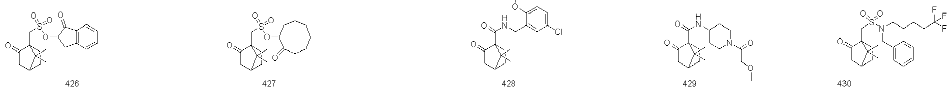

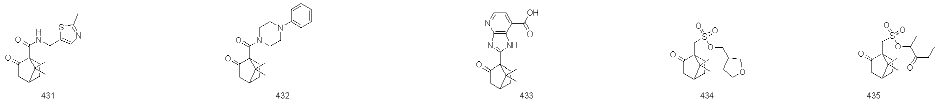

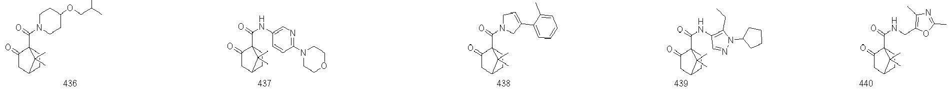

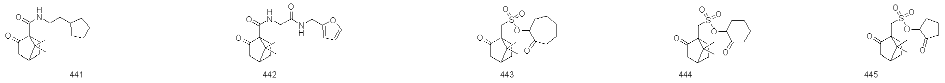

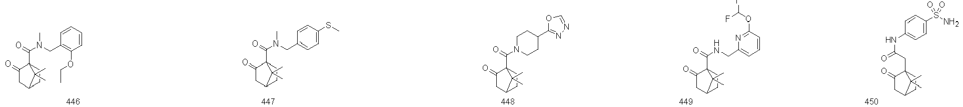

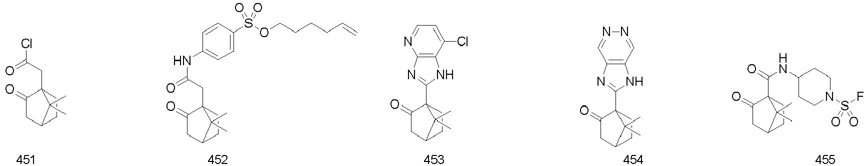

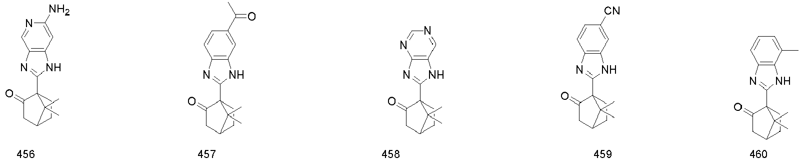

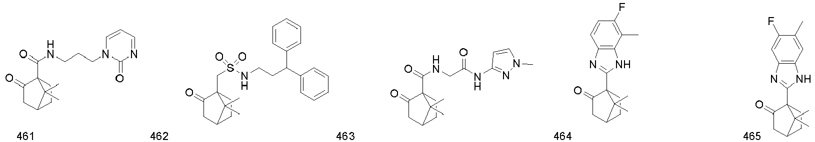

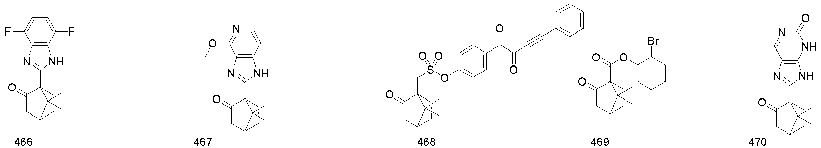

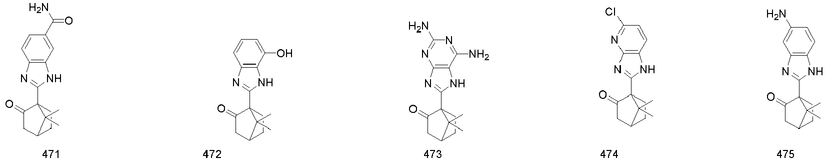

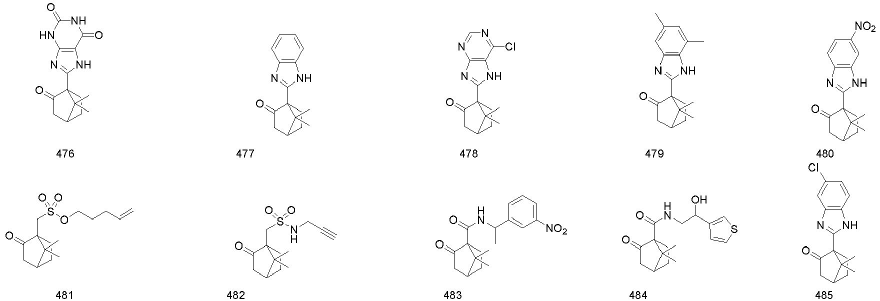

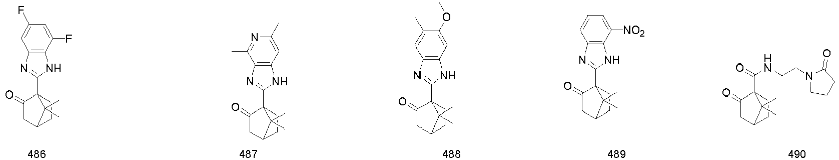

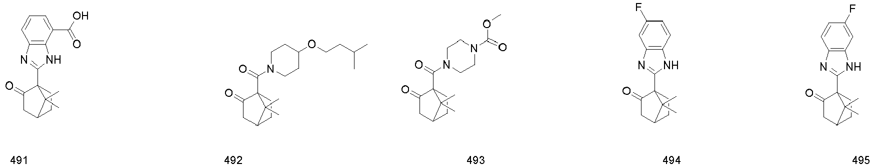

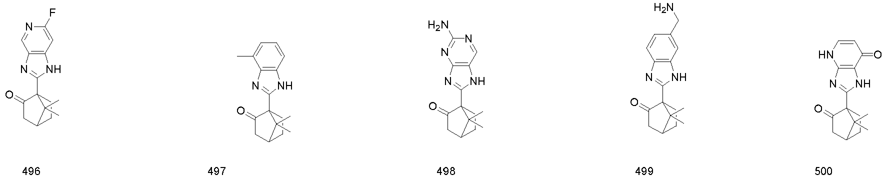

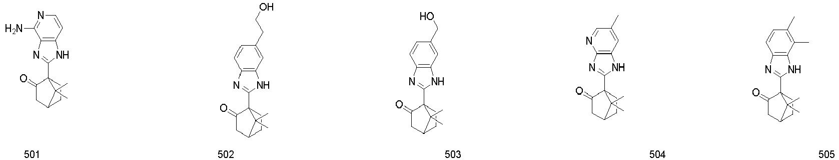

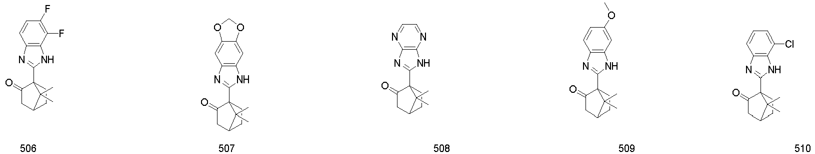

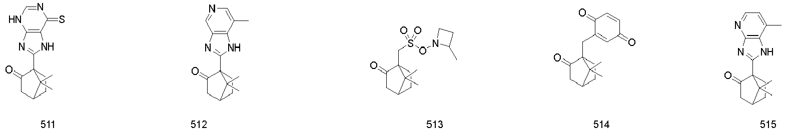

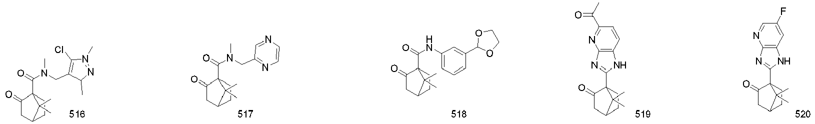

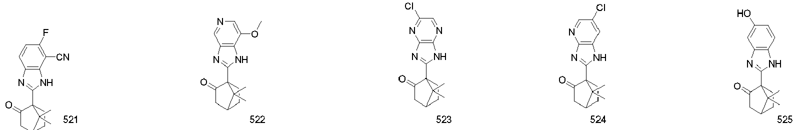

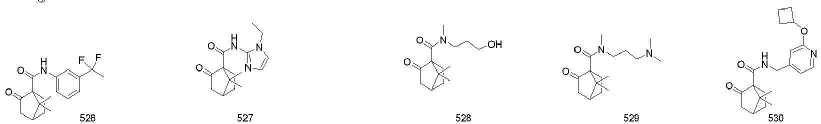

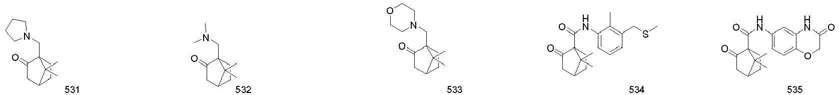

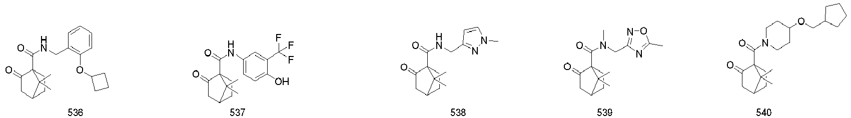

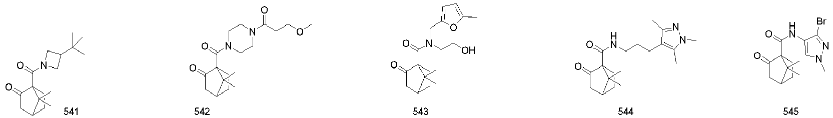

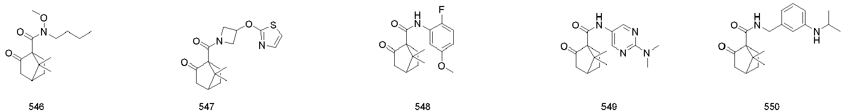

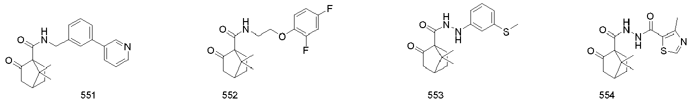

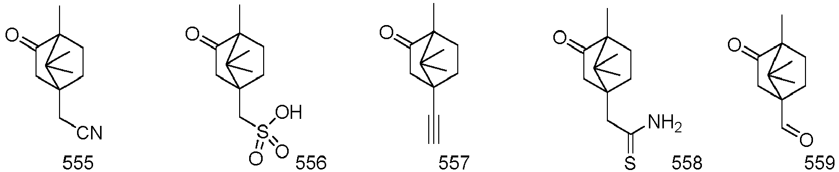

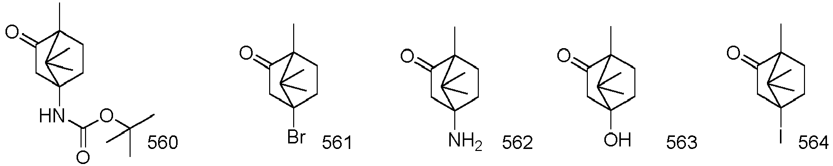

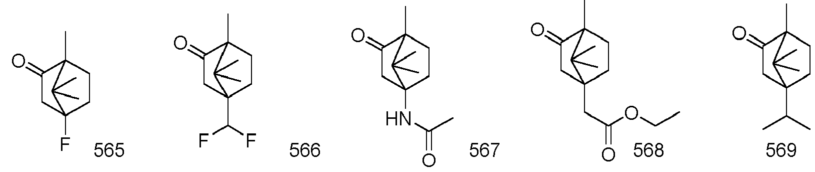

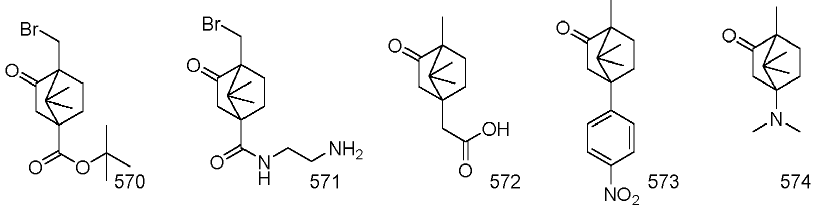

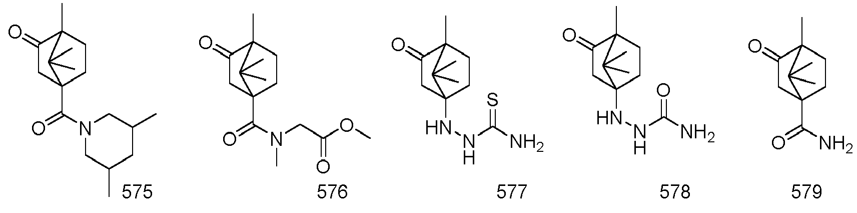

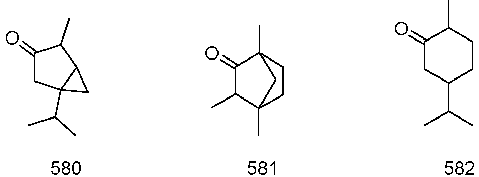

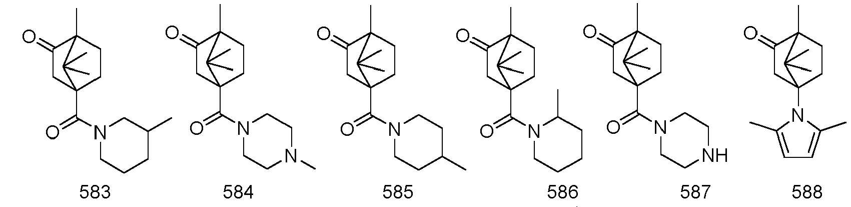

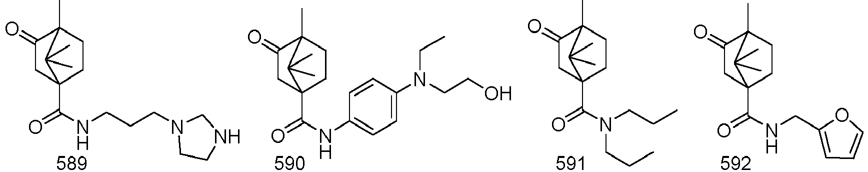

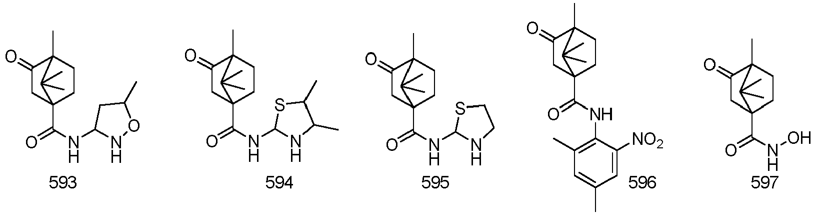

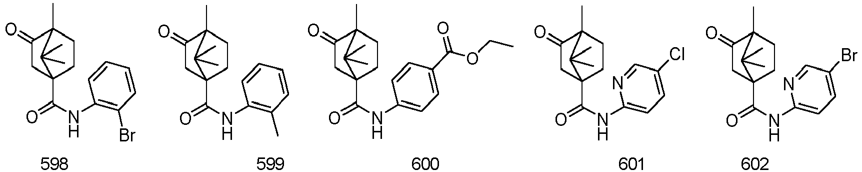

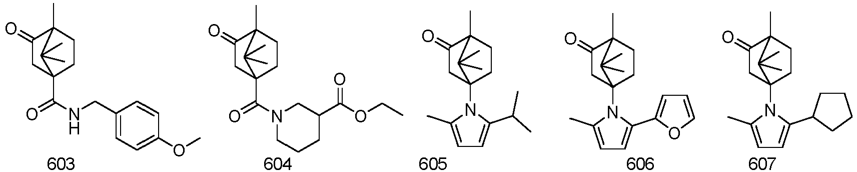

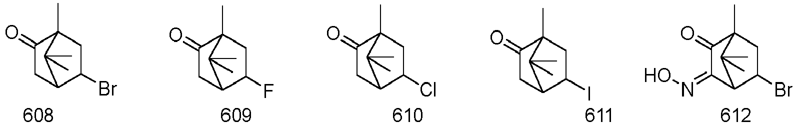

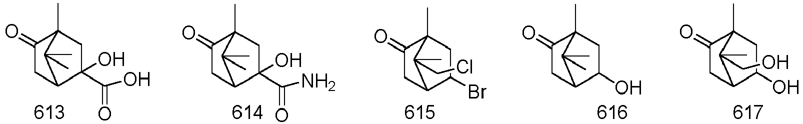

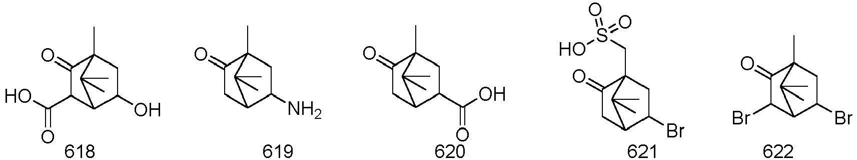

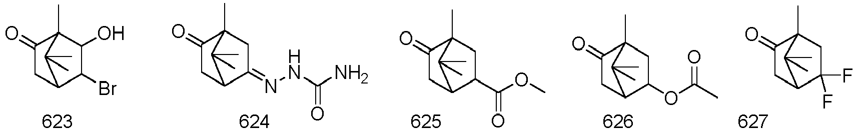

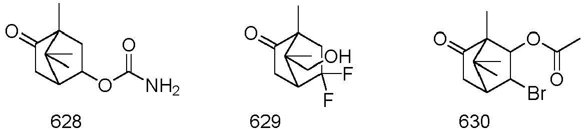

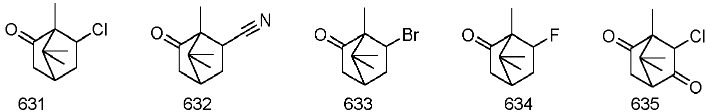

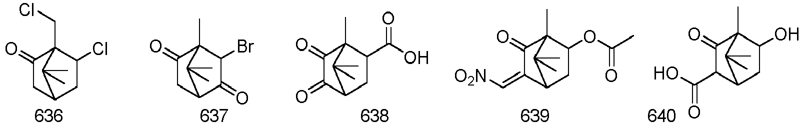

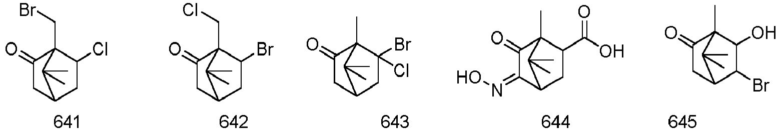

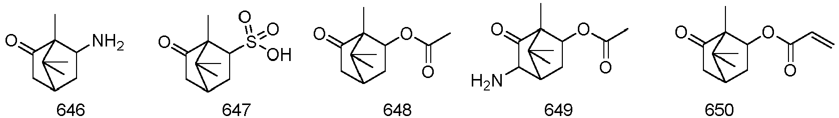

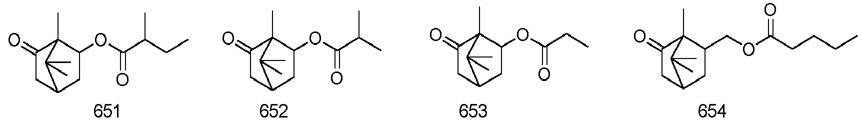

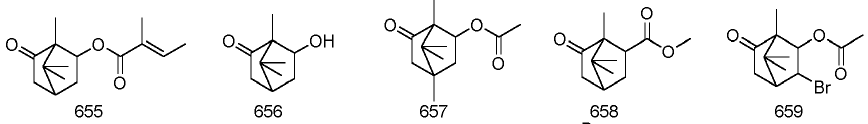

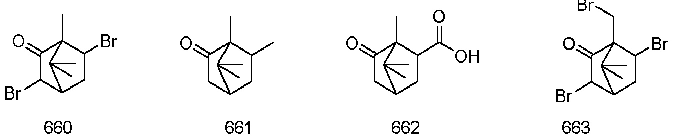

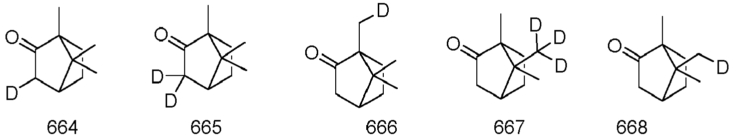

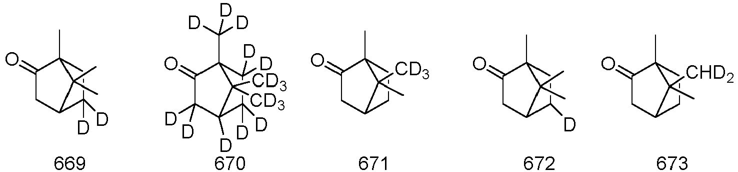

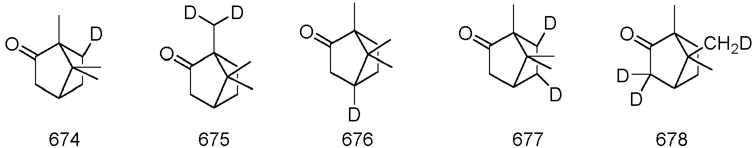

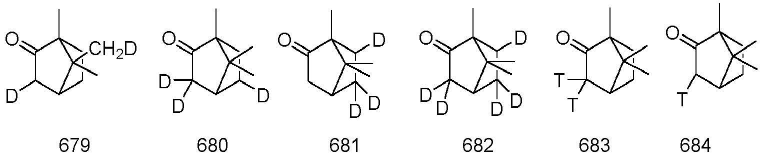

Further, said compound is selected from the group consisting of:

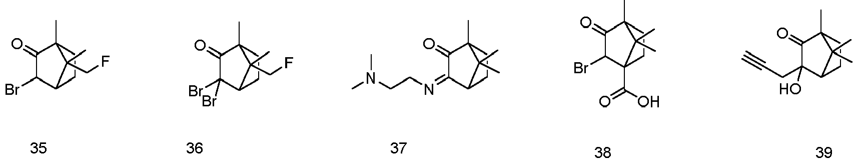

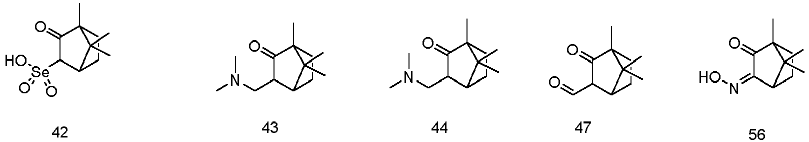

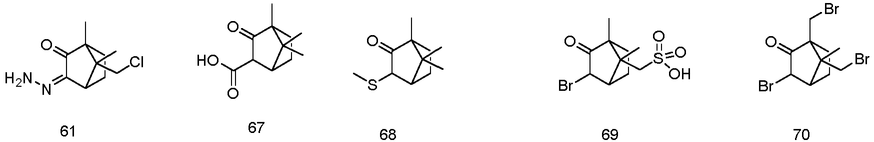

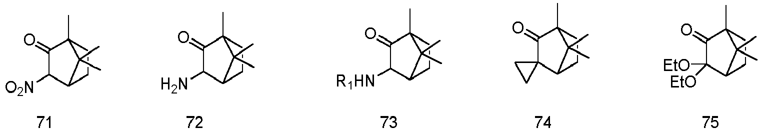

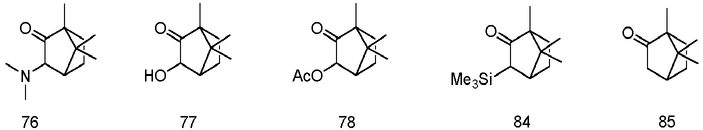

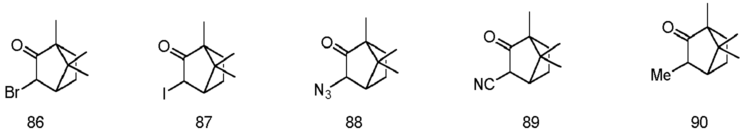

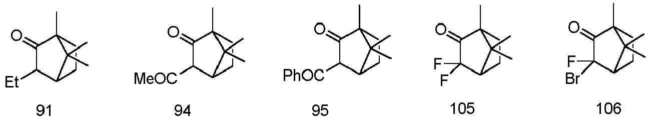

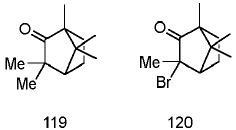

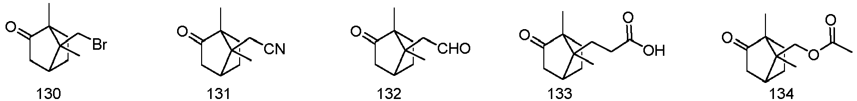

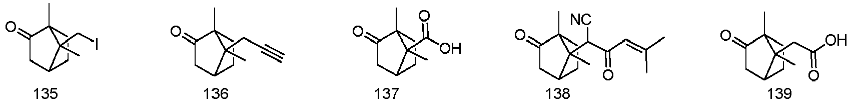

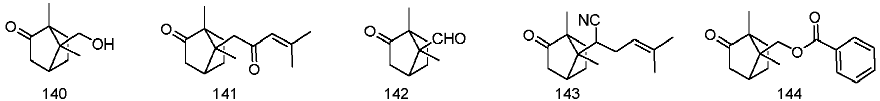

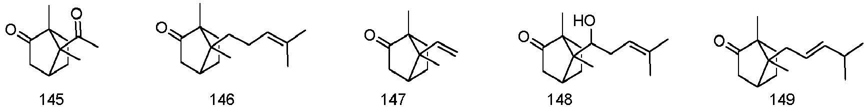

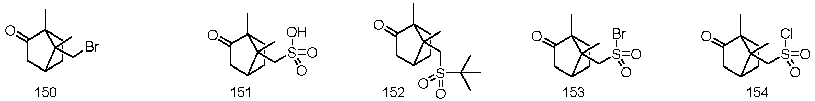

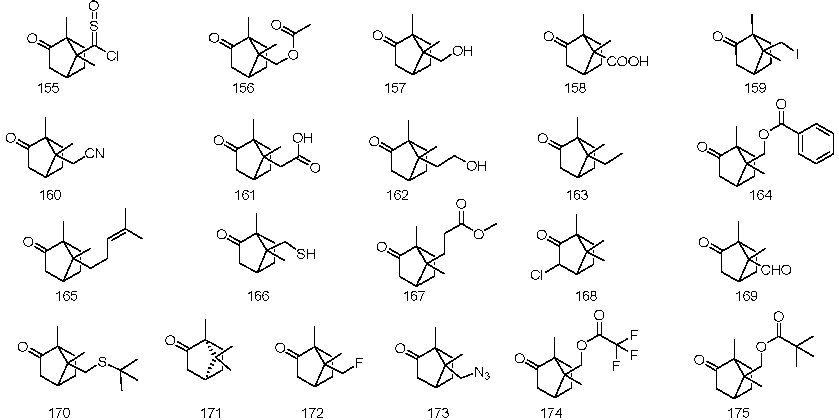

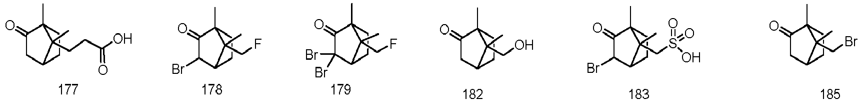

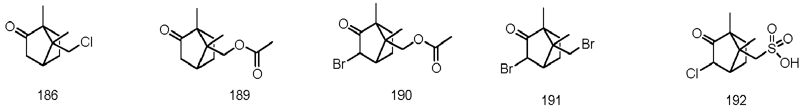

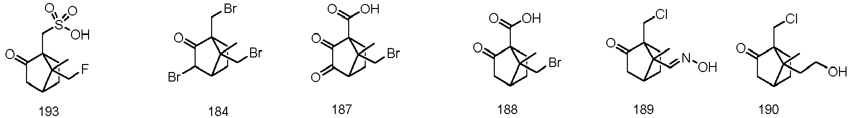

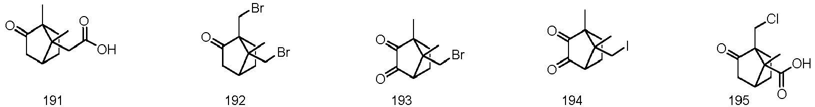

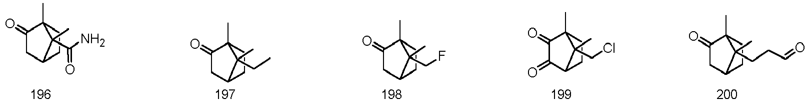

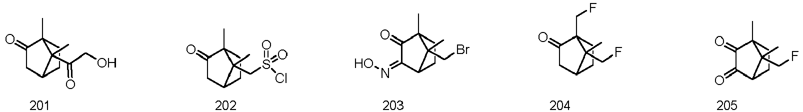

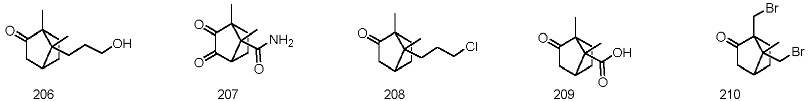

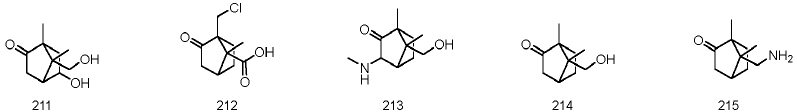

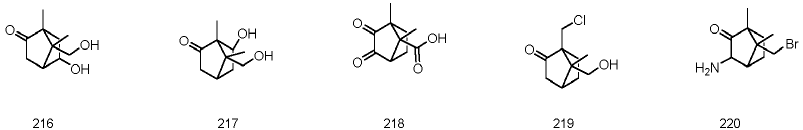

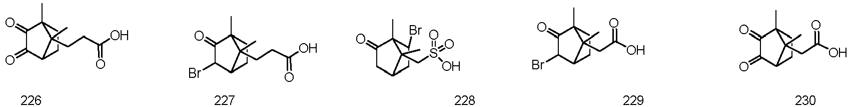

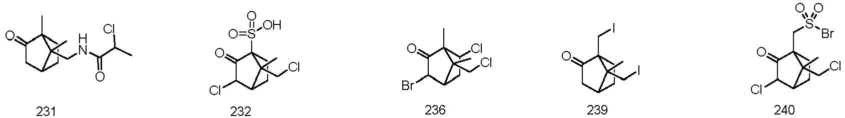

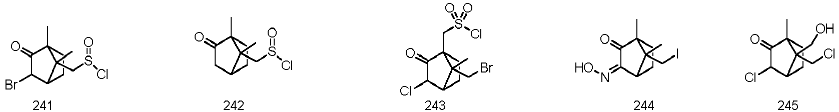

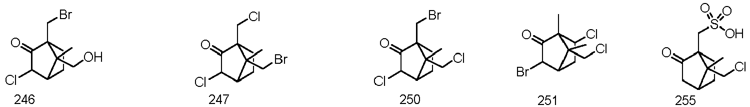

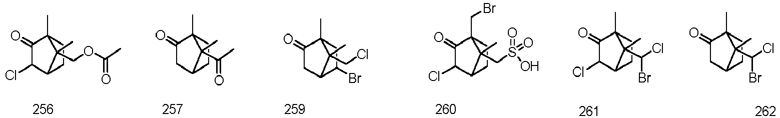

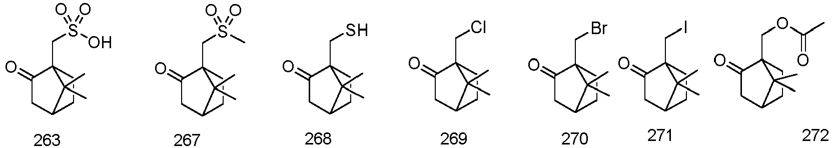

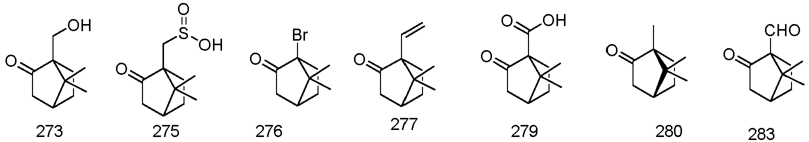

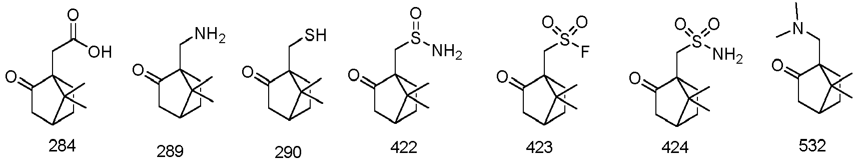

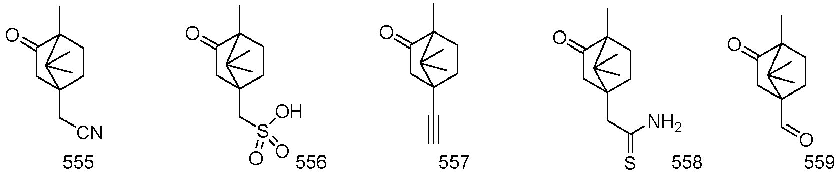

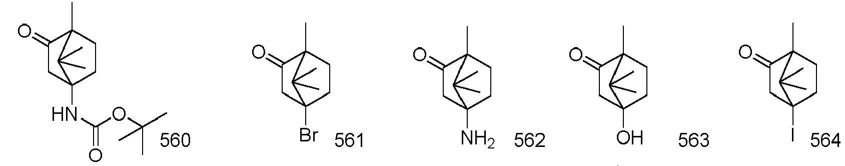

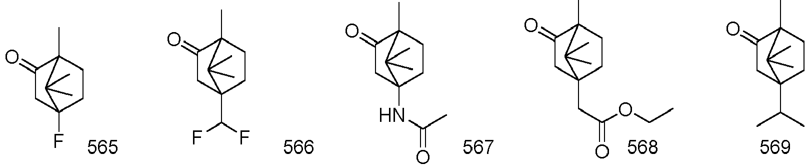

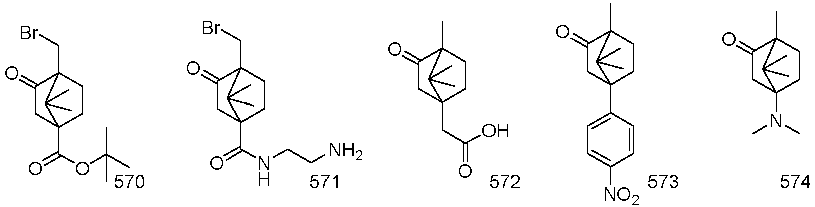

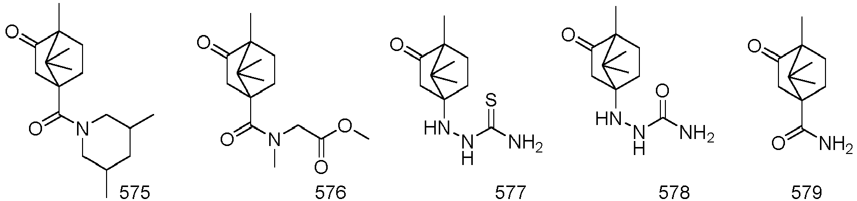

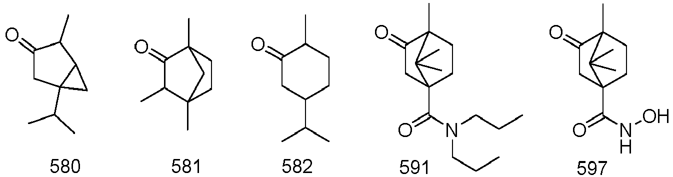

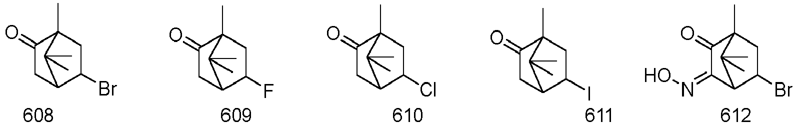

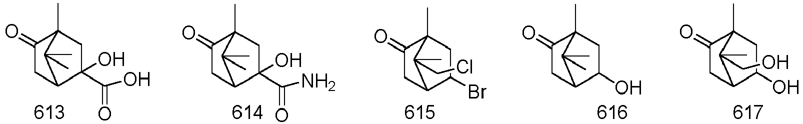

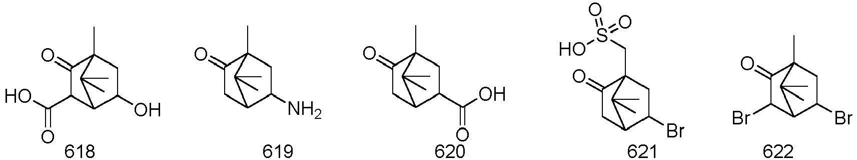

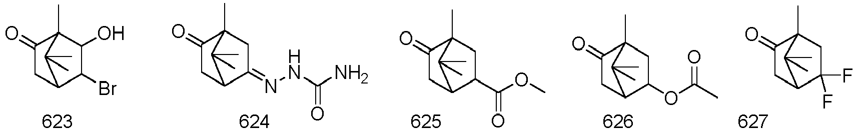

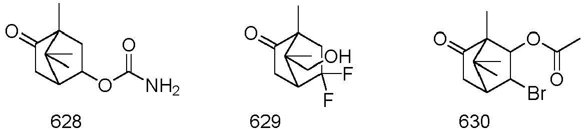

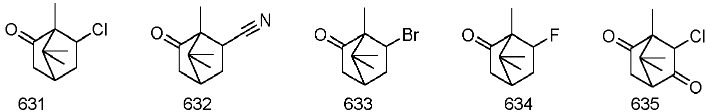

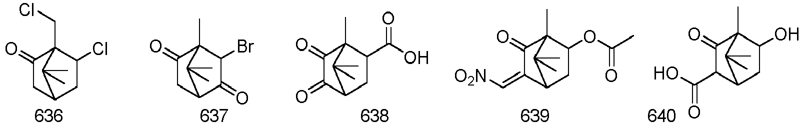

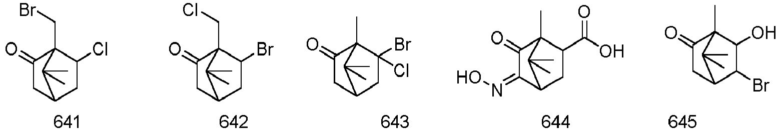

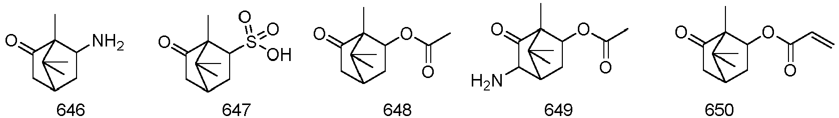

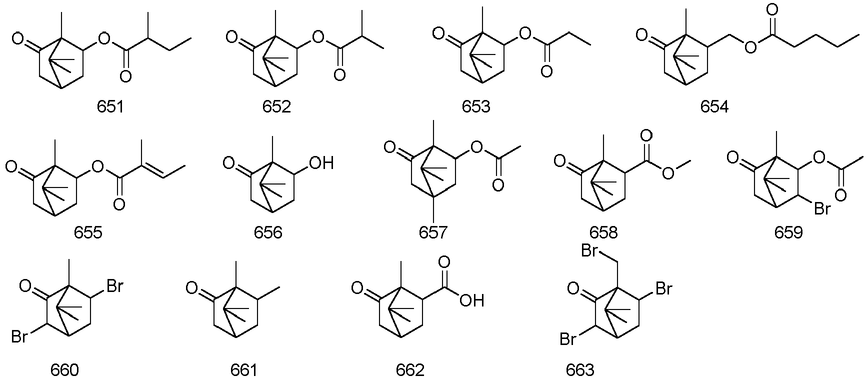

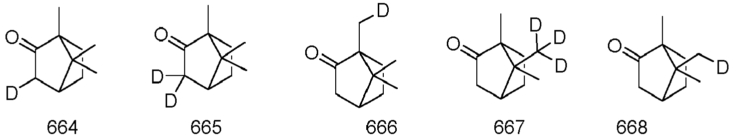

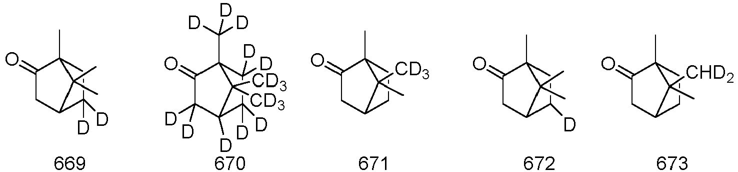

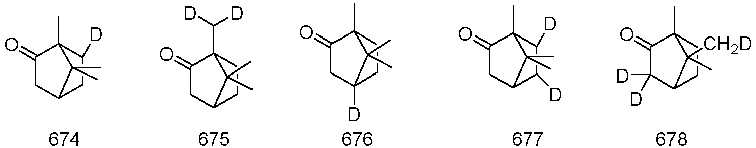

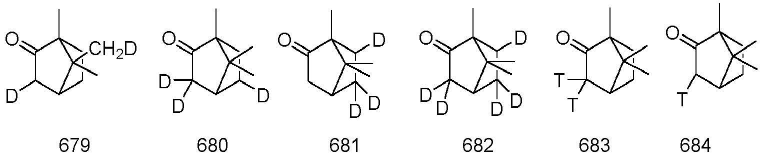

Further, said compound is selected from the group consisting of:

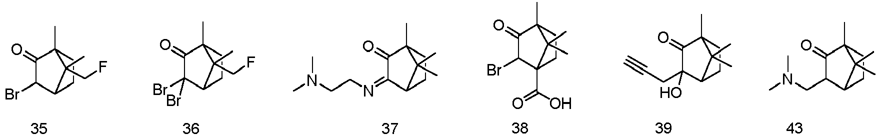

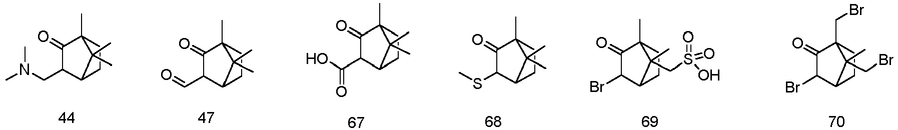

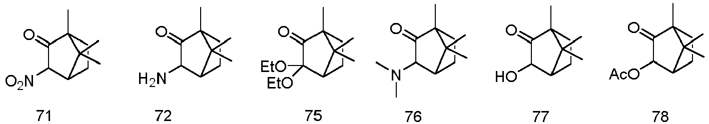

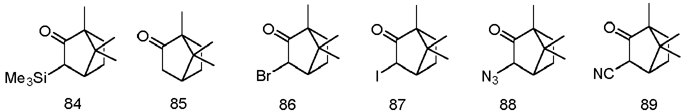

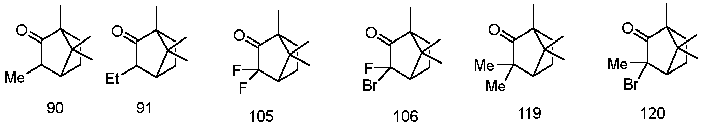

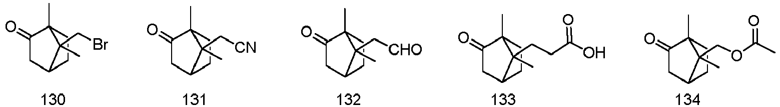

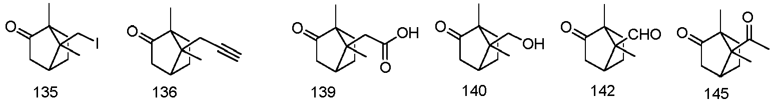

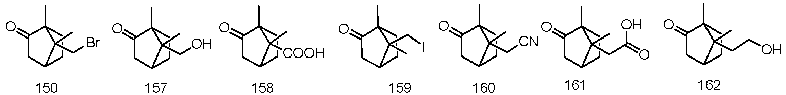

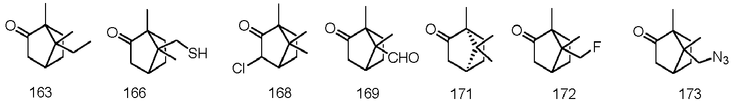

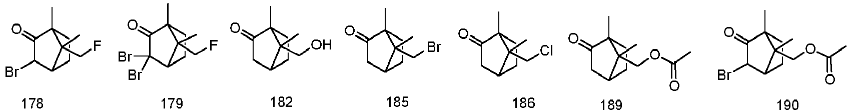

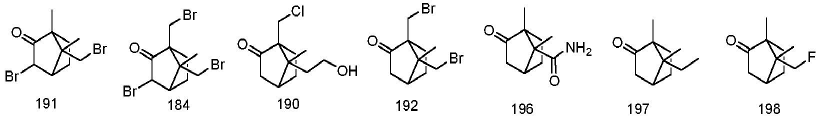

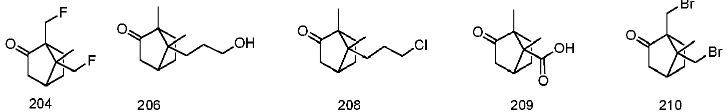

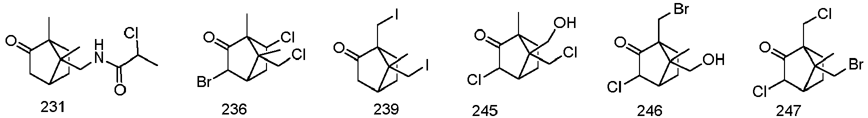

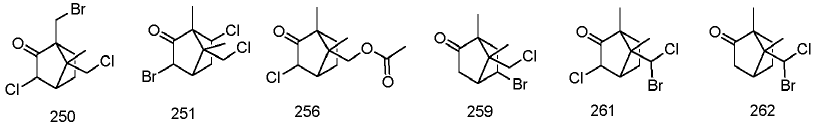

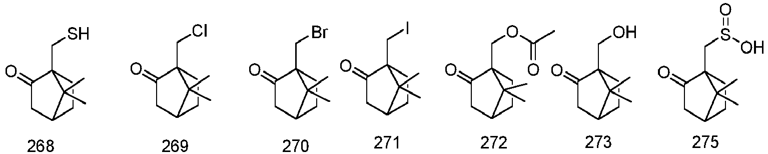

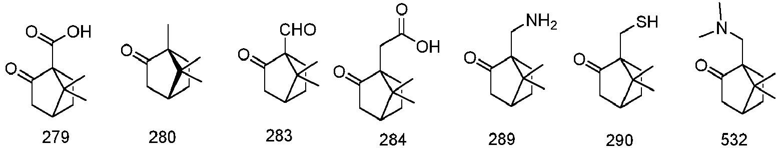

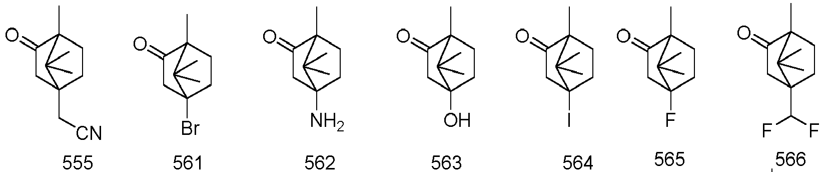

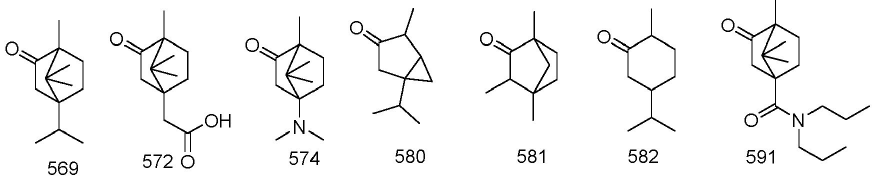

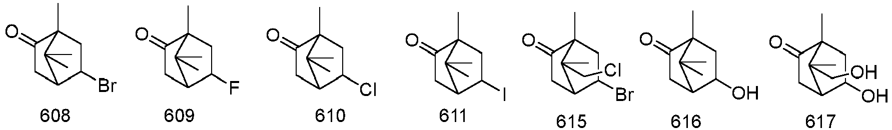

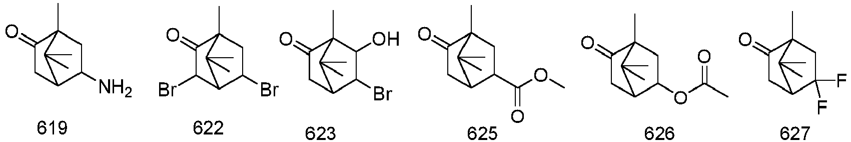

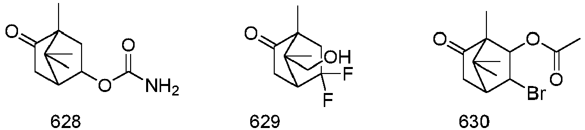

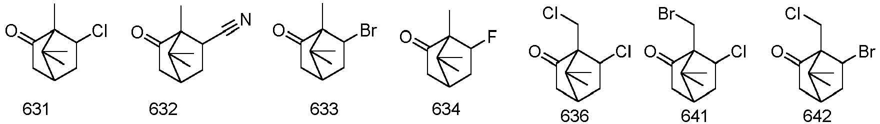

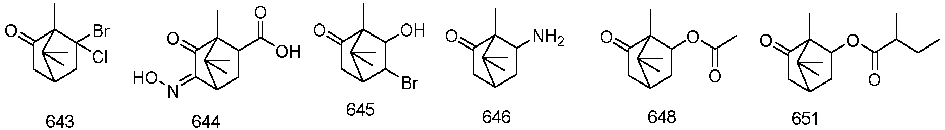

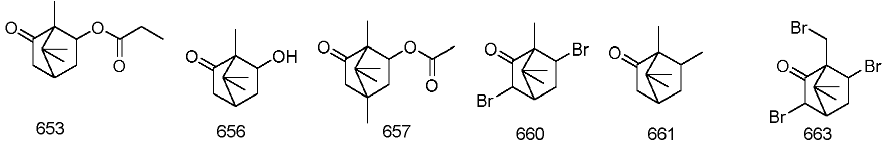

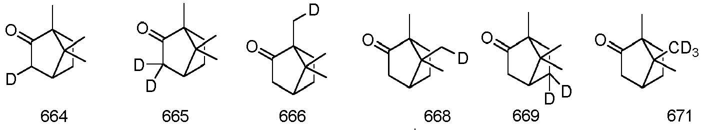

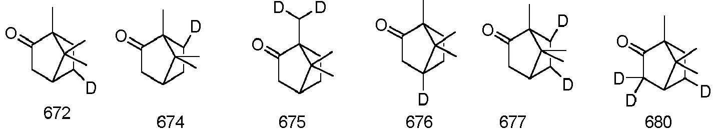

Further, the anesthetic and/or sedative antagonist is an antagonist of an anesthetic and/or sedative.

Further, the anesthetic and/or sedative antagonist can reverse the anesthetic and/or sedative state induced or maintained by anesthetics and/or sedative drugs.

Further, the anesthetic and/or sedative antagonist can reduce the duration of anesthesia and/or sedation.

Further, the anesthetics include a GABA_{A} receptor agonist.

Further, the anesthetic and/or sedative antagonist is a high affinity ligand of GABA_{A} receptor that can act as a competitive antagonist against GABA_{A} receptor agonists.

Further, the anesthetic is a general anesthetic.

Further, the general anesthesic include benzodiazepine drugs, substituted phenol drugs, imidazole drugs, GABA-like drugs, and phenylcyclohexylamine drugs.

Further, the benzodiazepine drugs include diazepam, midazolam, lorazepam, and remimazolam;
the substituted phenol drugs include propofol and ciprofol;
the imidazole drugs include etomidate or its derivatives;
the GABA-like drugs include γ-aminobutyric acid and γ-hydroxybutyric acid;
the phenylcyclohexylamine drugs include ketamine and fluoketamine.

Further, the anesthetic and/or sedative antagonist is a preparation prepared with the compounds, or stereoisomers thereof, or deuterated derivatives thereof, or metabolites thereof, or prodrugs thereof, or salts thereof, or solvates thereof, as the active ingredient, in combination with pharmaceutically acceptable excipients.

Further, the preparation is tablet, capsule, oral liquid, granule, pill, powder, injection or powder injection.

For the definition of terms used in the present invention: unless defined otherwise, the initial definition provided for the group or term herein applies to the group or term of the whole specification; for the terms that are not specifically defined herein, they should have the same meaning as commonly understood by one of ordinary skill in the art to which the disclosure belongs.

The minimum and the maximum for the content of carbon atoms in hydrocarbon groups are represented by prefixes, such as the prefix C_{a-b} alkyl indicates any alkyl having "a" to "b" carbon atoms. For example, C₁₋₅ alkyl means a straight or branched alkyl having 1-5 carbon atoms.

Herein, "substitution" means that one, two or more hydrogens in a molecule are substituted with other different atoms or molecules, including one, two or more substitutions on the same or different atoms in the molecule.

A "substituted or unsubstituted" group refers to a group that may not be further substituted or may be further substituted with one or more substituents. Unless otherwise specified, the substituent can be any substituent that can make the structure of the final compound be stable.

"Fused cycloalkyl" refers to a polycyclic cycloalkyl, in which two rings share two adjacent carbons.

"Fused heterocyclyl" refers to a polycyclic heterocyclyl, in which two rings share two adjacent carbons or heteroatoms.

Halogens are fluorine, chlorine, bromine, or iodine.

"A deuterated derivative" refers to the compound obtained by substituting one or more hydrogens in a compound with deuterium.

"A tritiated derivative" refers to a compound obtained by substituting one or more hydrogens in a compound with tritium.

"Salt" refers to an acidic and/or basic salt formed by combining a compound or its stereoisomer with inorganic and/or organic acids and/or bases, as well as zwitterionic salts (inner salts) and quaternary ammonium salts, such as alkyl ammonium salts. These salts can be directly obtained during the final separation and purification of the compound. It can also be obtained by mixing a compound or its stereoisomer with a certain amount of acid or base (such as equivalency). These salts may form precipitates in a solution and be collected by filtration, recovered after evaporation of solvents, or prepared by freeze-drying after reaction in an aqueous medium.

The salt mentioned in the present invention can be a compound's hydrochloride, sulfate, citrate, benzenesulfonate, hydrobromate, hydrofluorate, phosphate, acetate, propionate, succinate, oxalate, malate, succinate, fumarate, maleate, tartrate, or trifluoroacetate.

In the present invention, it is first discovered that the cyclic ketone compounds represented by formula I have potent antagonism on general anesthesia and sedation. The experimental results have shown that the compound of the present invention has a good antagonistic action on general anesthesia induced by various general anesthetics (including propofol, etomidate, remimazolam, etc.), and can obviously shorten the time for mice to recover their righting reflex after general anesthesia, as well as the time for mice to resume normal walking. Moreover, the compound of the present invention can also have antagonistic activities on the maintenance of general anesthesia by continuous infusion of the general anesthetic propofol.

The present invention provides a new choice for antagonists of anesthetic and/or sedative drugs, especially those of general anesthetics, with broad application prospects.

Obviously, based on the above content of the present invention, according to the common technical knowledge and the conventional means in the field, without department from the above basic technical spirits, other various modifications, alternations, or changes can further be made.

With reference to the following specific examples of the embodiments, the above content of the present invention is further illustrated. But it should not be construed that the scope of the above subject matter of the present invention is limited to the following examples. The techniques realized based on the above content of the present invention are all within the scope of the present invention.

### Examples

The raw materials and equipment used in the present invention are known products obtained by purchasing those commercially available.

The compounds used in the following examples can be obtained by purchasing commercially available products or synthesized using known methods in the prior art.

### Example 1. Antagonism of each compound against different general anesthetics

### 1. Main reagents and drugs

### (1) Main reagents

Propofol injection (batch number: 2010083, Xi'an Libang), etomidate injection (purity >98%), remimazolam (purity >98%), medium and long chain fat emulsion injection.

### (2) Test drug

The preparation method: A suitable amount of the compound to be tested was weighed, to which was added a suitable amount of medium and long chain fat emulsion injection with a pipette, and thus a 50 mmol/L solution of the test compound was prepared. After filtering with a 0.22 µm microporous membrane, the solution was immediately used.

### 2. Experimental methods

Male ICR mice weighing 25 g ± 1 g were randomly divided into 8 groups, with 8 mice in each group. Propofol (22.11 mg/kg, 1.5x ED₅₀), etomidate (4.48 mg/kg, 2× ED₅₀), and remimazolam (57.34 mg/kg, 1.5× ED₅₀) were injected into the tail vein for anesthesia induction, with an injection time of 15 seconds. After maintaining the disappearance of the mouse righting reflex for 30 seconds, the solution of each test compound (50 mg/kg, using medium and long chain fat emulsion injection as solvent) or equal volume of medium and long chain fat emulsion injection was respectively injected, and the total anesthesia time of the mice was observed and recorded. Among them, the total anesthesia time refers to the time from the disappearance of the mouse righting reflex to the recovery of the mouse righting reflex.

### 3. Experimental results

The results are shown in Table 1.

**Table 1. The activities of test drugs on antagonizing the anesthesia effect of general anesthetics.**

| General anesthetics | Test drugs | The average of total anesthesia time (min) |
|---|---|---|
| Propofol | Fat emulsion | 7.97±0.85 |
| | Compound **67** | 2.14±0.32 |
| | Compound **68** | 4.05±0.15 |
| | Compound **85** | 0.47±0.21 |
| | Compound **86** | 1.56±0.21 |
| | Compound **130** | 1.79±0.09 |
| | Compound **140** | 4.13±0.20 |
| | Compound **150** | 1.26±0.17 |
| | Compound **157** | 2.52±0.21 |
| | Compound **163** | 1.51±0.51 |
| | Compound **171** | 0.42±0.32 |
| | Compound **269** | 2.15±0.98 |
| | Compound **270** | 2.49±0.15 |
| | Compound **273** | 1.43±0.43 |
| | Compound **280** | 0.48±0.29 |
| | Compound **563** | 2.62±0.18 |
| | Compound **565** | 2.40±0.89 |
| | Compound **580** | 3.12±0.12 |
| | Compound **581** | 2.22±0.27 |
| | Compound **582** | 1.39±0.08 |
| | Compound **608** | 4.08±0.34 |
| | Compound **616** | 2.70±0.63 |
| | Compound **631** | 3.30±0.79 |
| | Compound **633** | 2.04±0.63 |
| | Compound **661** | 2.89±0.64 |
| Etomidate | Fat emulsion | 7.77±0.65 |
| | Compound **68** | 2.11±0.54 |
| | Compound **85** | 0.46±0.25 |
| | Compound **87** | 1.56±0.45 |
| | Compound **135** | 1.72±0.50 |
| | Compound **139** | 2.64±0.89 |
| | Compound **140** | 2.05±0.33 |
| | Compound **157** | 2.08±0.32 |
| | Compound **163** | 2.17±0.60 |
| | Compound **171** | 1.97±0.07 |
| | Compound **270** | 2.50±0.99 |
| | Compound **280** | 0.73±0.25 |
| | Compound **561** | 2.03±0.61 |
| | Compound **565** | 0.87±0.93 |
| | Compound **580** | 1.36±0.84 |
| | Compound **581** | 1.07±0.32 |
| | Compound **582** | 1.27±0.83 |
| | Compound **616** | 1.15±0.83 |
| | Compound **626** | 2.05±0.54 |
| | Compound **633** | 0.96±0.92 |
| | Compound **643** | 2.80±0.67 |
| Remimazolam | Fat emulsion | 4.57±1.32 |
| | Compound **85** | 0.45±0.21 |
| | Compound **86** | 1.49±0.62 |
| | Compound **87** | 0.84±0.78 |
| | Compound **139** | 1.29±0.63 |
| | Compound **140** | 1.16±0.92 |
| | Compound **163** | 0.77±0.02 |
| | Compound **168** | 0.86±0.42 |
| | Compound **171** | 0.73±0.57 |
| | Compound **269** | 1.39±0.45 |
| | Compound **270** | 1.01±0.05 |
| | Compound **280** | 0.69±0.38 |
| | Compound **572** | 0.85±0.37 |
| | Compound **580** | 0.69±0.25 |
| | Compound **581** | 1.32±0.31 |
| | Compound **582** | 1.49±0.21 |
| | Compound **610** | 1.32±0.06 |
| | Compound **616** | 0.94±0.30 |
| | Compound **631** | 0.70±0.32 |
| | Compound **661** | 0.55±0.76 |
| Ketamine | Fat emulsion | 7.26±0.40 |
| | Compound **70** | 3.49±0.40 |
| | Compound **85** | 3.01±0.45 |
| | Compound **104** | 2.67±0.30 |
| | Compound **120** | 3.72±0.24 |
| | Compound **171** | 3.64±0.89 |
| | Compound **262** | 3.92±0.83 |
| | Compound **280** | 3.08±0.67 |
| | Compound **565** | 3.46±0.15 |
| | Compound **633** | 3.35±0.90 |
| | Compound **664** | 3.20±0.77 |

Experiments had shown that for general anesthetics such as propofol, etomidate, and remimazolam, fat emulsion hadn't had antagonistic effects on the anesthesia; however, the compounds listed in the table could all have antagonistic activities on general anesthesia, whose potency was proven by a significant reduction in the total anesthesia time in mice. The compound provided in the present invention could antagonize the anesthesia effect of general anesthetics and provide faster awakening speed.

### Example 2. Antagonism of compounds against continuous infusion of general anesthetics

### 1. Main reagents and drugs

### (1) Main reagents

Propofol injection (batch number: 2010083, Xi'an Libang), medium and long chain fat emulsion injection.

### (2) Test drug

The preparation method was the same as that of Example 1.

### 2. Experimental method

24 male SD rats with a body weight range of 250-300 g were randomly divided into 4 groups, with 6 rats in each group. Continuous anesthesia induction was performed using propofol (22.11 mg/kg, 1.5x ED₅₀), and then maintained at a rate of 1 mg/(kg·min) for 30 min. After drug withdrawal, compound **85** at doses of 10 mg/kg, 20 mg/kg, and 50 mg/kg, as well as 50 mg/kg of the test compound solutions (using medium and long chain fat emulsion injection as solvent) or equal volume of medium and long chain fat emulsion injection was immediately injected, respectively, and the anesthesia recovery time of the mice after stopping the medication was observed and recorded. Among them, the anesthesia recovery time refers to the time from the cessation of propofol infusion to the recovery of the righting reflex in mice.

### 3. Experimental results

The results are shown in Table 2.

**Table 2. The activities of test drugs on antagonizing the general anesthesia effect maintained by continuous infusion of propofol.**

| Dosage of test drug | Test drug | The average of awakening time (min) |
|---|---|---|
| - | Fat emulsion | 8.98±1.41 |
| 50 mg/kg | Compound **67** | 3.04±0.18 |
| 10 mg/kg | Compound **85** | 4.11±1.22 |
| 20 mg/kg | Compound **85** | 3.68±1.12 |
| 50 mg/kg | Compound **85** | 0.36±0.98 |
| 50 mg/kg | Compound **130** | 3.41±0.52 |
| 50 mg/kg | Compound **163** | 1.33±0.79 |
| 50 mg/kg | Compound **171** | 0.34±0.89 |
| 50 mg/kg | Compound **269** | 3.29±0.81 |
| 50 mg/kg | Compound **280** | 0.40±0.92 |
| 50 mg/kg | Compound **565** | 2.58±0.56 |
| 50 mg/kg | Compound **580** | 2.76±0.20 |
| 50 mg/kg | Compound **581** | 4.66±0.60 |
| 50 mg/kg | Compound **582** | 0.96±0.23 |
| 50 mg/kg | Compound **610** | 2.72±0.36 |
| 50 mg/kg | Compound **631** | 2.85±0.58 |
| 50 mg/kg | Compound **633** | 2.64±0.19 |
| 50 mg/kg | Compound **643** | 1.01±0.29 |
| 50 mg/kg | Compound **661** | 2.39±0.75 |

From the experimental results, it was indicated that compound **85** provided in the present invention could effectively reverse the anesthesia maintenance action induced by continuous infusion of propofol, and compound **85** could dose-dependently shorten the anesthesia recovery time of rats. In addition, the compounds listed in the table could reverse the anesthesia maintenance effect of continuous infusion of propofol, which was manifested as a shortened average awakening time in mice.

In summary, the present invention provided the use of cyclic ketone compounds represented by formula I in the manufacture of anesthetic and/or sedative antagonists. The present invention first disclosed that the cyclic ketone compounds had good antagonistic effects on general anesthesia and sedation. The experimental results had shown that the compound of the present invention had a good antagonistic action on general anesthesia induced by various general anesthetics (including propofol, etomidate, remimazolam, etc.), and could obviously shorten the time for mice to recover their righting reflex after general anesthesia, as well as the time for mice to resume normal walking. Moreover, the compound of the present invention could also have antagonistic activities on the maintenance of general anesthesia by continuous infusion of the general anesthetic propofol. The present invention provided a new choice for antagonists of anesthetic and/or sedative drugs, especially those of general anesthetics, with broad application prospects.

## Claims

1. The use of compounds represented by formula I, or stereoisomers thereof, or deuterated derivatives thereof, or tritiated derivatives thereof, or metabolites thereof, or prodrugs thereof, or salts thereof, or solvates thereof in the manufacture of anesthetic and/or sedative antagonists: 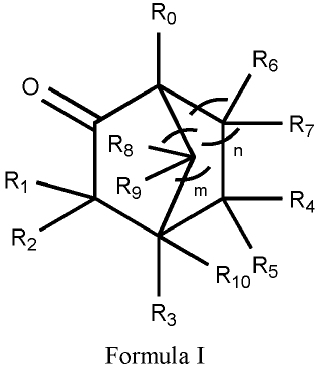
wherein, n is 0 or 1;
m is 0 or 1; when m is 1, R₁₀ is absent;
R₀ is selected from the group consisting of L₀, L₁, L₂, and Rₓ₁;
L₀, L₁, and L₂ are each independently selected from absence, and substituted or unsubstituted following groups: NHCO, NHCS, CO, COO, OCO, OCOO, C(SO), O, S, SO, SO₂, NH, CH=N, CONH, NH(CS)S(CH₂), C₁₋₃ alkylene, C₂₋₄ alkenylene, and C₂₋₄ alkynylene;
Rₓ₁ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₁ and R₂ are each independently selected from the group consisting of L₃, L₄, L₅, Rₓ₂, =NL₅Rₓ₂, and =C(L₆Rₓ₃)(L₇Rₓ₄); or R₁ and R₂ are linked to form a ring;
L₃, L₄, L₅, L₆, and L₇ are each independently selected from absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₂, R_{X3}, and Rₓ₄ are each independently selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₃ and R₁₀ are each independently selected from the group consisting of L₈, L₉, L₁₀, and Rₓ₅ ;
L₈, L₉, and L₁₀ are each independently selected from the group consisting of absence, as well as substituted or unsubstituted following groups: NHCO, NHCS, CO, COO, OCO, OCOO, C(SO), O, S, SO, SO₂, NH, CH=N, CONH, NH(CS)S(CH₂), and C₁₋₃ alkylene;
Rₓ₅ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₄, R₅, R₆, and R₇ are each independently selected from the group consisting of L₁₁, L₁₂, L₁₃, Rₓ₆, =NL₁₃Rₓ₆, =C(L₁₄Rₓ₇)(L₁₅Rₓ₈); L₁₁, L₁₂, L₁₃, L₁₄, and L₁₅ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₆, Rₓ₇, and Rₓ₈ are each independently selected from the group consisting of hydrogen, deuterium, tritium, halogen, aldehyde group, CN, N₃, NO₂, OAc, as well as substituted or unsubstituted following groups: OSiH₃, OOSiH₃, SO₃H, Sift, CS₂H, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
or, R₁₀ and R₅ are linked to form a ring;
R₈ and R₉ are each independently selected from L₁₆, L₁₇, L₁₈, and Rₓ₉;
L₁₆, L₁₇, and L₁₈ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₉ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl.

2. The use according to claim 1, **characterized in that** said compound is those represented by formula II: 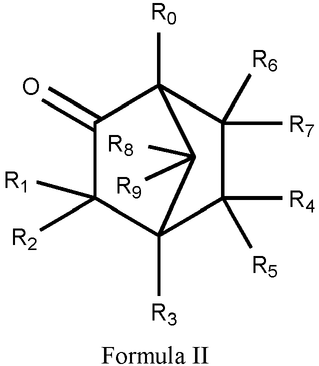
wherein, R₀ is selected from L₀, L₁, L₂, and Rₓ₁;
L₀, L₁, and L₂ are each independently selected from the group consisting of absence, as well as substituted or unsubstituted following groups: NHCO, NHCS, CO, COO, OCO, OCOO, C(SO), O, S, SO, SO₂, NH, CH=N, CONH, NH(CS)S(CH₂), C₁₋₃ alkylene, C₂₋₄ alkenylene, and C₂₋₄ alkynylene;
Rₓ₁ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₁ and R₂ are each independently selected from the group consisting of L₃, L₄, L₅, Rₓ₂, =NL₅Rₓ₂, and =C(L₆Rₓ₃)(L₇Rₓ₄); or R₁ and R₂ are linked to form a ring;
L₃, L₄, L₅, L₆, and L₇ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₂, R_{X3}, and R_{X4} are each independently selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₃ is selected from L₈, L₉, L₁₀, and Rₓ₅;
L₈, L₉, and L₁₀ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₅ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₄, R₅, R₆, R₇ are each independently selected from the group consisting of L₁₁, L₁₂, L₁₃, Rₓ₆, =NL₁₃Rₓ₆, and =C(L₁₄Rₓ₇)( L₁₅Rₓ₈); L₁₁, L₁₂, L₁₃, L₁₄, and L₁₅ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₆, Rₓ₇, Rₓ₈ are each independently selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl;
R₈ and R₉ are each independently selected from the group consisting of L₁₆, L₁₇, L₁₈, and Rₓ₉;
L₁₆, L₁₇, and L₁₈ are each independently selected from the group consisting of absence, as well as substituted or unsubstituted following groups: NHCO, NHCS, CO, COO, OCO, OCOO, C(SO), O, S, SO, SOz, NH, CH=N, CONH, NH(CS)S(CH₂), and C₁₋₃ alkylene;
Rₓ₉ is selected from the group consisting of hydrogen, deuterium, tritium, halogen, aldehyde group, CN, N₃, NO₂, OAc, as well as substituted or unsubstituted following groups: OSiH₃, OOSiH₃, SO₃H, SiH₃, CS₂H, CSNH₂, NH₂, COOH, SH, SeO₂H, OH, N=NH, =O, CH=NH, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl.

3. The use according to claim 2, **characterized in that** said compound is those represented by formula III: 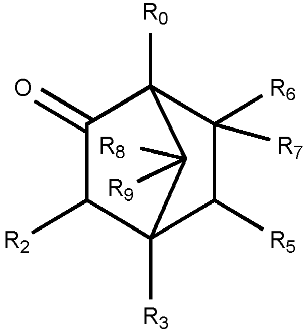 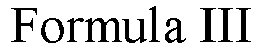
wherein, R₀ is selected from the group consisting of hydrogen, deuterium, tritium, C₁₋₅ alkyl, C₁₋₅ alkyl substituted with 1-3 Rₐ, C₁₋₅ alkoxy, C₁₋₅ alkoxy substituted with 1-3 Rₐ, C₂₋₆ alkenyl, C₂₋₆ alkynyl, saturated or unsaturated heterocyclyl, saturated or unsaturated cycloalkyl, fused cycloalkyl, and fused heterocyclyl; Rₐ is each independently selected from the group consisting of deuterium, tritium, halogen, and hydroxyl;
R₂ is selected from the group consisting of hydrogen, deuterium, tritium, halogen, COORₑ, SRₑ, ORₑ, C₁₋₅ alkyl, =NRₕ₁, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl; Rₑ is selected from the group consisting of hydrogen, deuterium, tritium, C₁₋₅ alkyl; Rₕ₁ is selected from the group consisting of hydroxyl and C₁₋₅ alkyl;
R₃ is selected from the group consisting of hydrogen, deuterium, tritium, hydroxyl, halogen, C₁₋₅ alkyl, L_{f}COOR_{f}, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl; L_{f} is absence or C₁₋₃ alkylene; Rf is selected from the group consisting of hydrogen, deuterium, tritium, and C₁₋₅ alkyl;
R₅ is selected from the group consisting of hydrogen, deuterium, tritium, halogen, hydroxyl, OCOR_{b}, C₁₋₅ alkyl, C₁₋₅ alkoxy, C₂₋₆ alkenyl, C₂₋₆ alkynyl, saturated or unsaturated heterocyclyl, saturated or unsaturated cycloalkyl, fused cycloalkyl, and fused heterocyclyl; R_{b} is C₁₋₅ alkyl;
R₆ and R₇ are each independently selected from the group consisting of hydrogen, deuterium, tritium, halogen, C₁₋₅ alkyl, COOR_{g}, C₁₋₅ alkoxy, C_{2~6} alkenyl, C_{2~6} alkynyl, saturated or unsaturated heterocyclyl, saturated or unsaturated cycloalkyl, fused cycloalkyl, and fused heterocyclyl; R_{g} is selected from the group consisting of hydrogen, deuterium, tritium, and C₁₋₅ alkyl;
R₈ and R₉ are each independently selected from the group consisting of hydrogen, deuterium, tritium, C₁₋₅ alkyl, C₁₋₆ alkyl substituted with 1-3 Rₐ, C₁₋₅ alkoxy, C₁₋₅ alkoxy substituted with 1-3 Rₐ, C₂₋₆ alkenyl, C₂₋₆ alkynyl, saturated or unsaturated heterocyclyl, saturated or unsaturated cycloalkyl, fused cycloalkyl, and fused heterocyclyl; R_{c} is each independently selected from the group consisting of halogen, COOR_{d}, hydroxyl, C₁₋₅ alkyl, deuterium, and tritium; R_{d} is selected from the group consisting of hydrogen, deuterium, tritium, and C₁₋₅ alkyl.

4. The use according to claim 3, **characterized in that** said compound is those represented by formula IV: 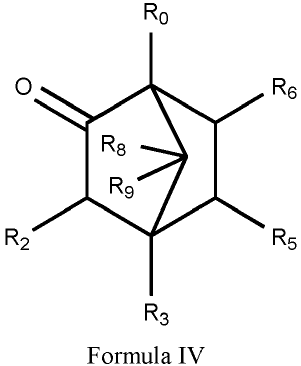
R₀ is selected from the group consisting of methyl, and methyl substituted with 1-3 Rₐ; Rₐ is each independently selected from the group consisting of deuterium, halogen, and hydroxyl;
R₂ is selected from the group consisting of hydrogen, halogen, COOH, SCH₃, methyl, and =N-OH;
R₃ is selected from the group consisting of hydrogen, hydroxyl, halogen, C₁₋₃ alkyl, and L_{f}COOH; L_{f} is methylene;
R₅ is selected from the group consisting of hydrogen, halogen, hydroxyl, OCOR_{b}; R_{b} is methyl;
R₆ is selected from the group consisting of hydrogen, halogen, methyl, and COOH;
R₈ and R₉ are each independently selected from the group consisting of hydrogen, methyl, and methyl substituted with 1-3 Rₐ; R_{c} is each independently selected from the group consisting of halogen, COOH, hydroxyl, methyl, and deuterium.

5. The use according to claim 1, **characterized in that** said compound is selected from the group consisting of

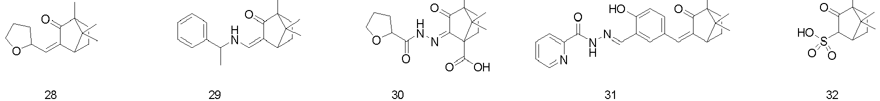

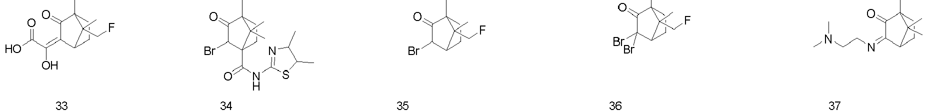

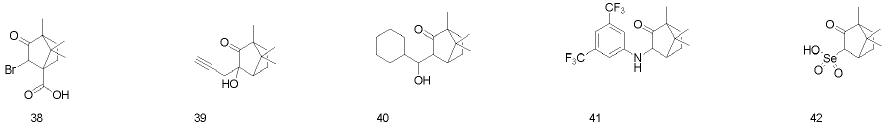

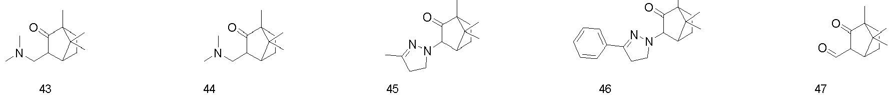

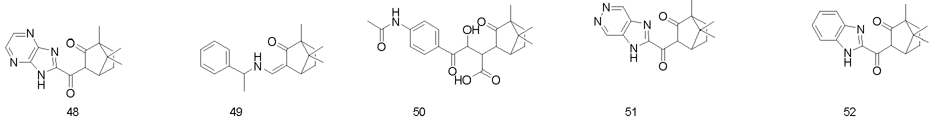

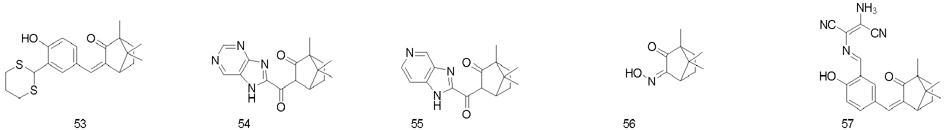

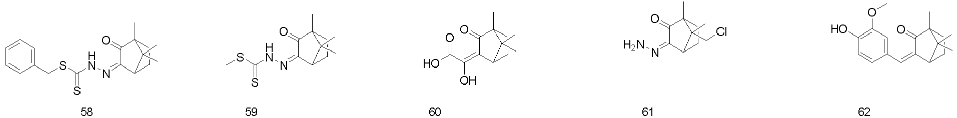

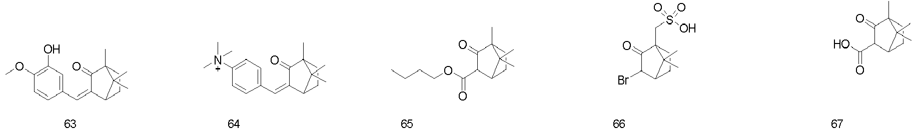

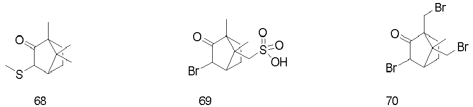

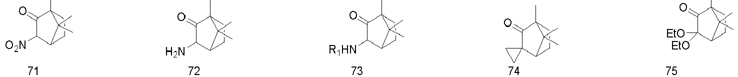

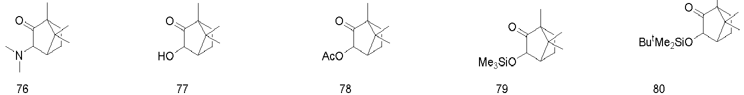

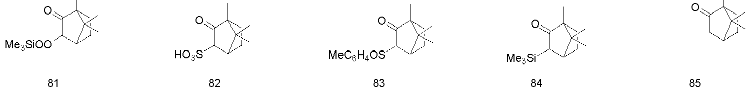

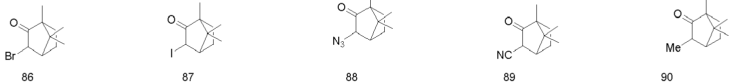

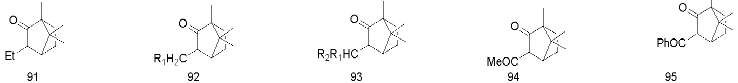

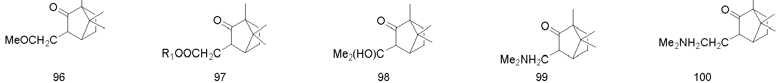

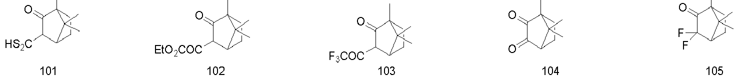

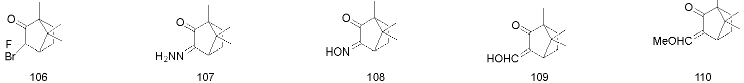

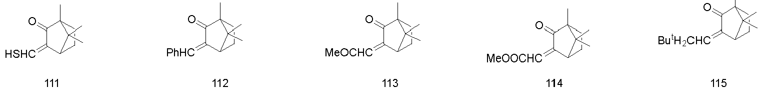

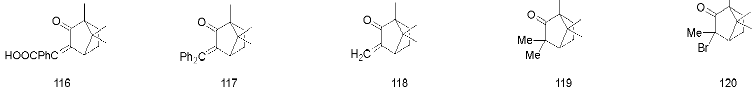

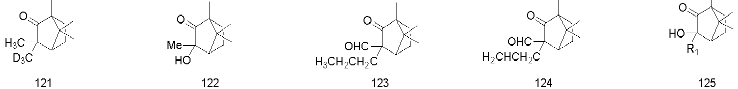

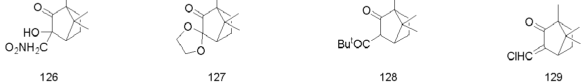

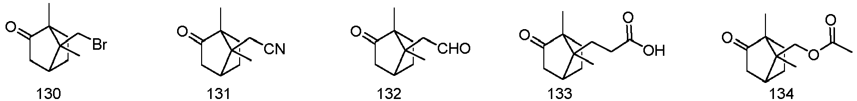

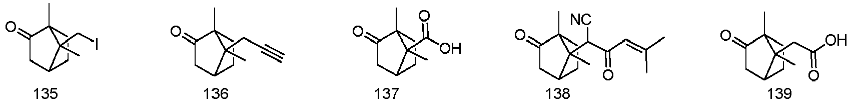

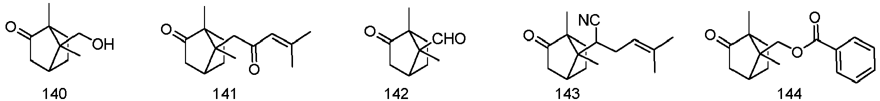

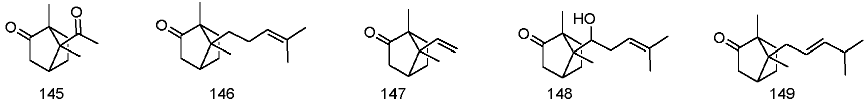

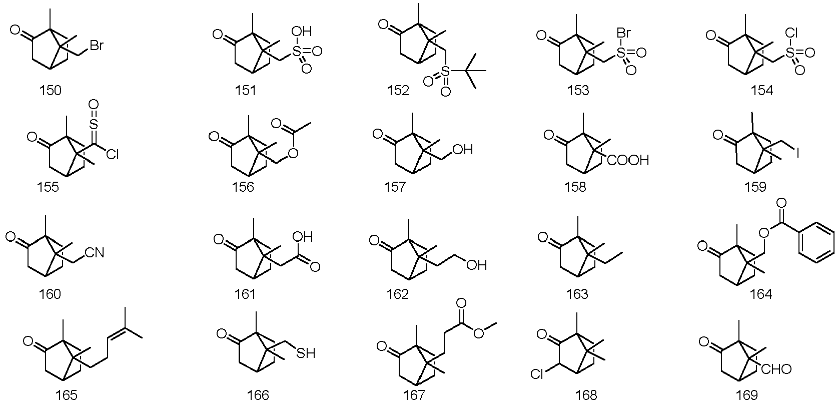

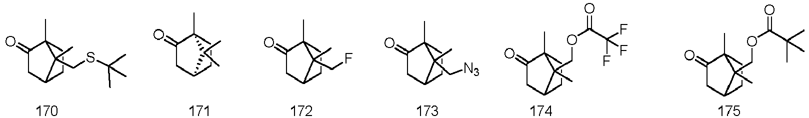

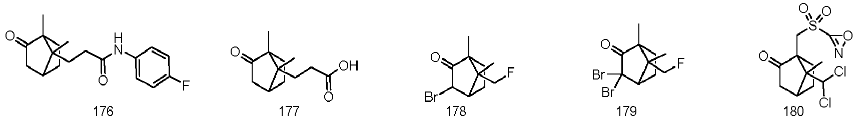

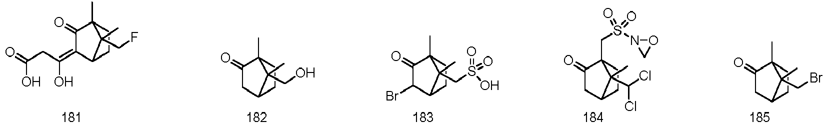

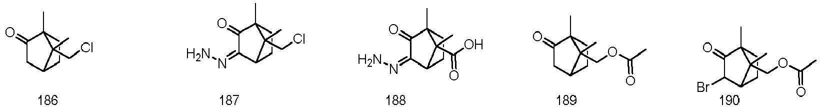

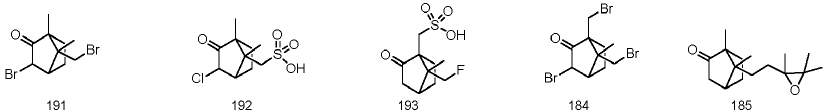

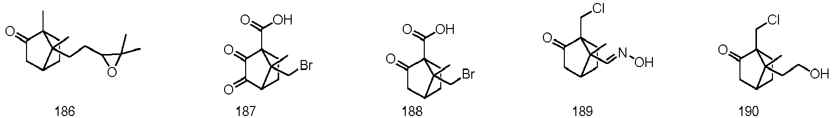

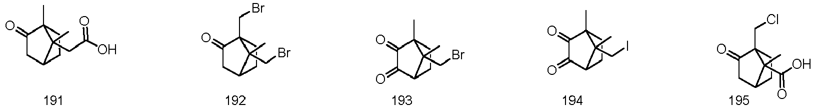

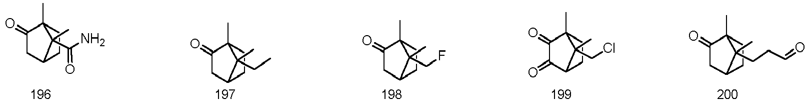

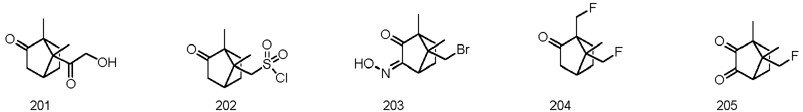

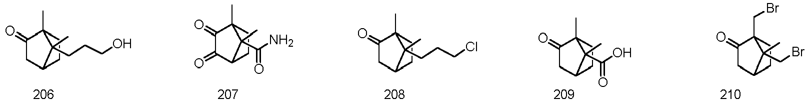

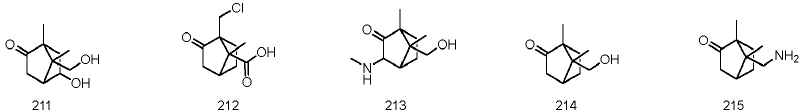

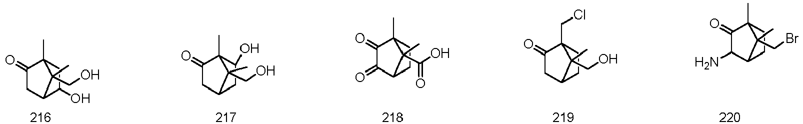

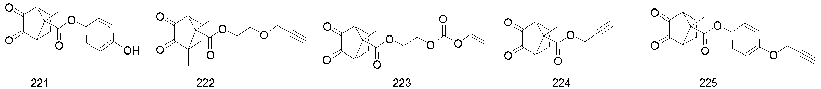

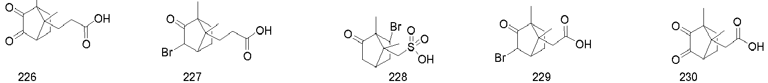

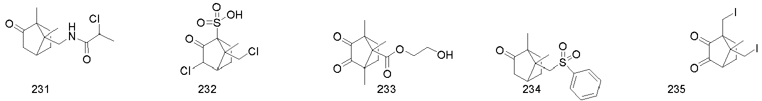

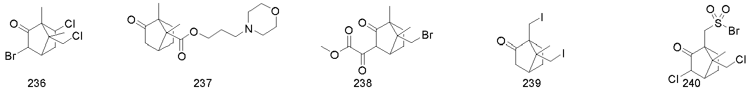

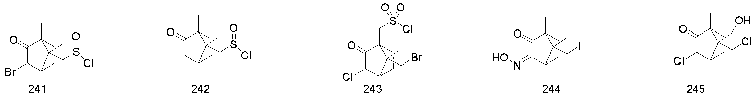

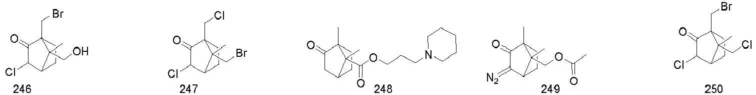

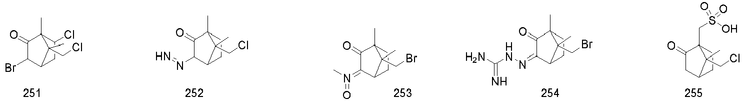

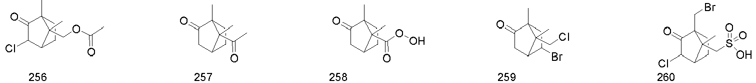

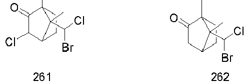

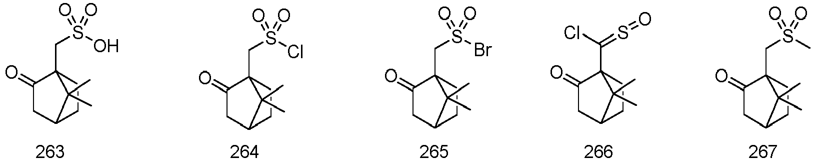

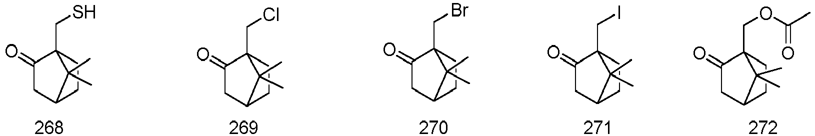

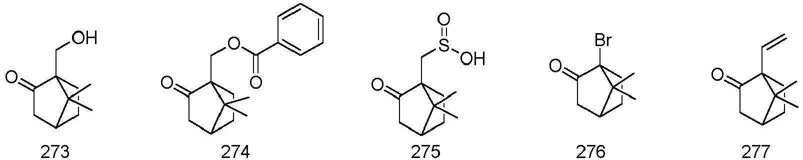

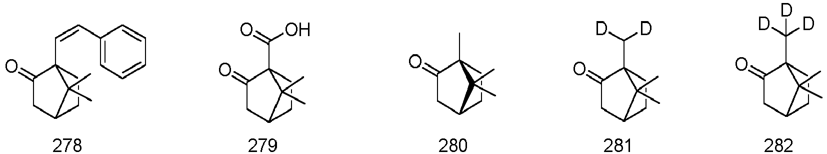

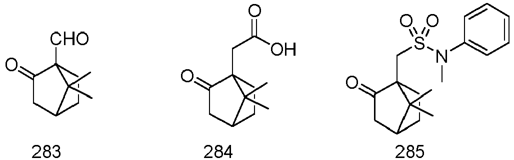

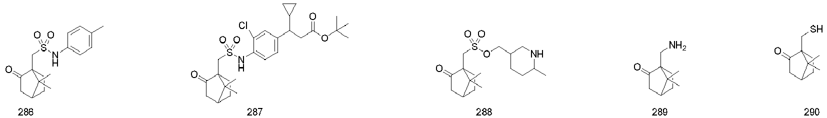

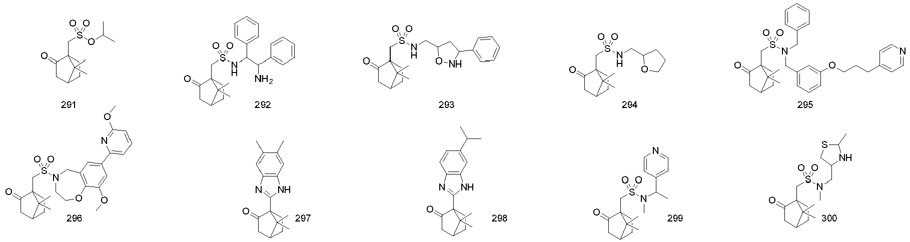

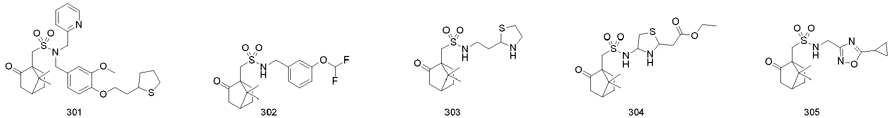

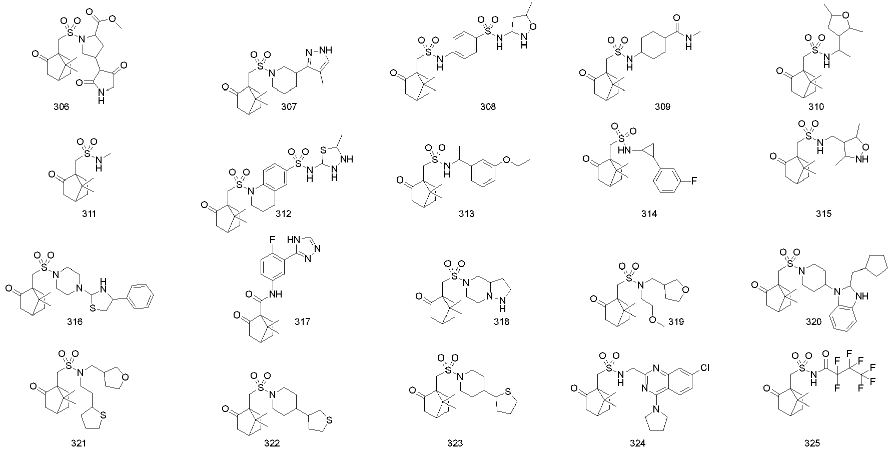

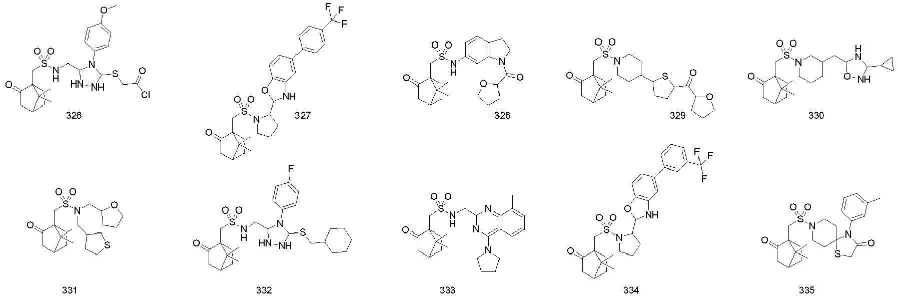

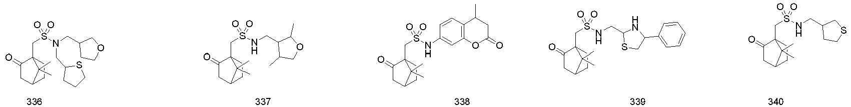

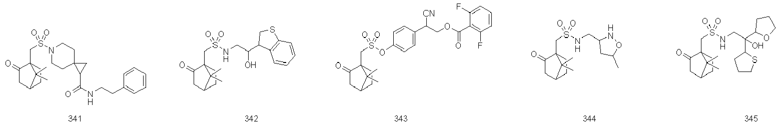

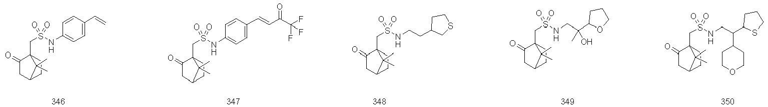

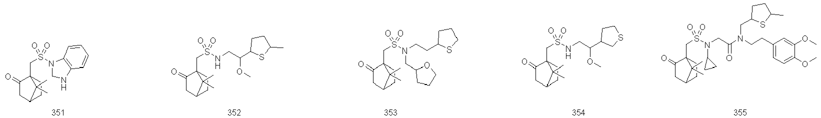

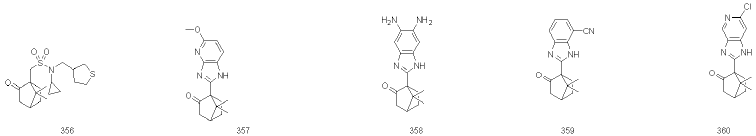

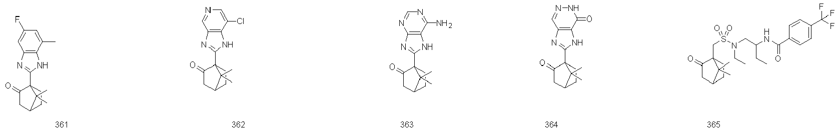

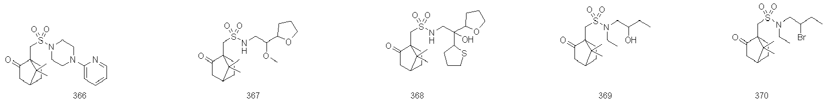

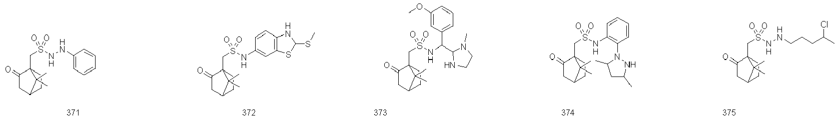

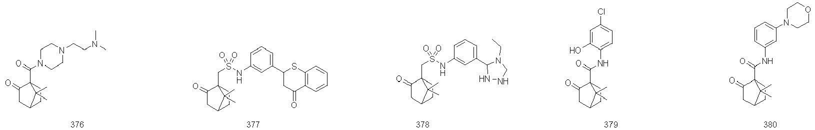

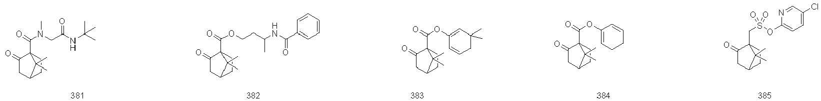

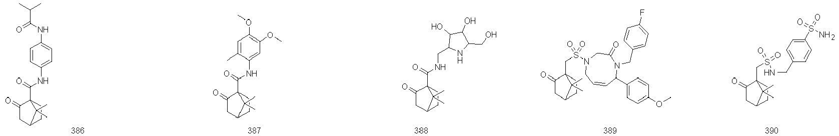

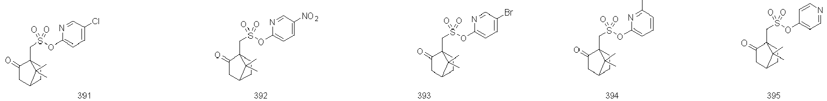

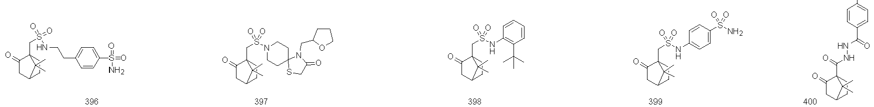

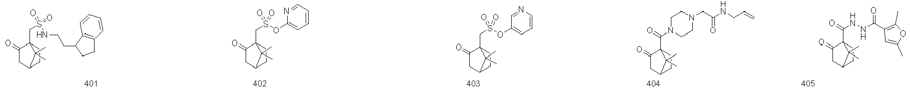

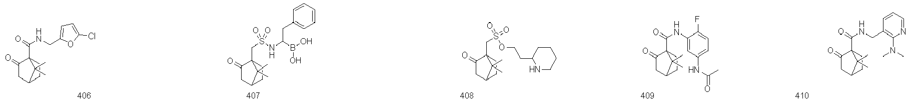

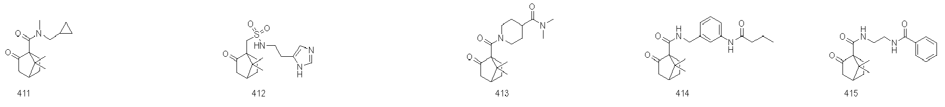

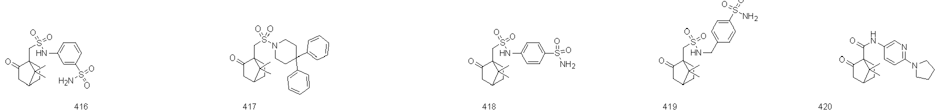

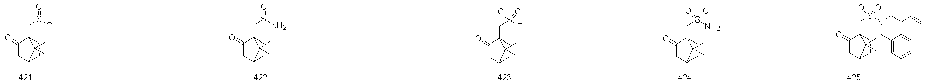

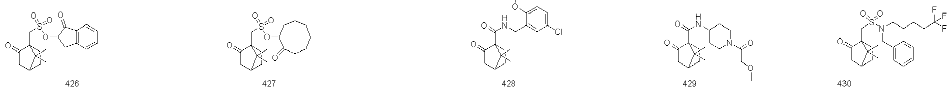

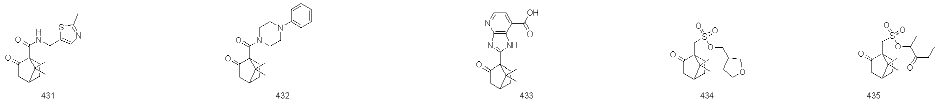

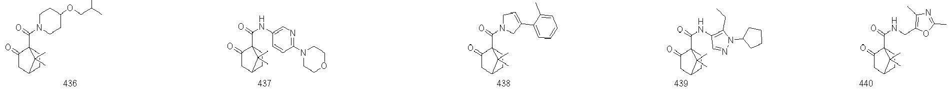

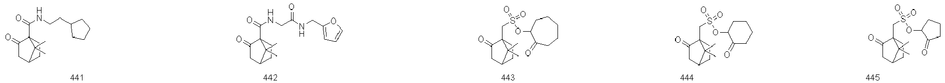

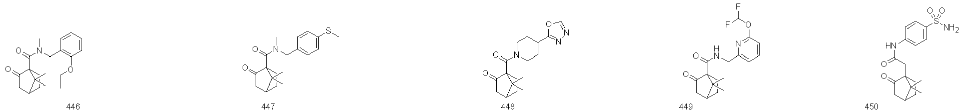

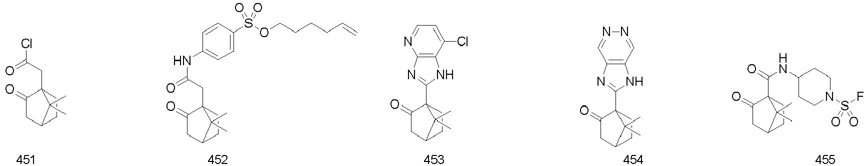

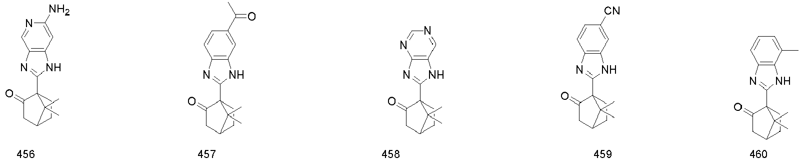

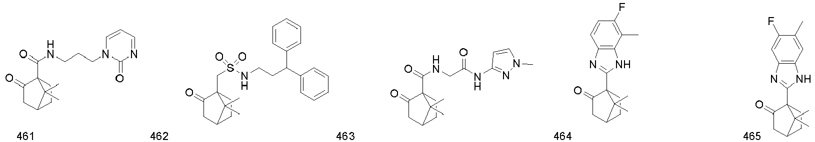

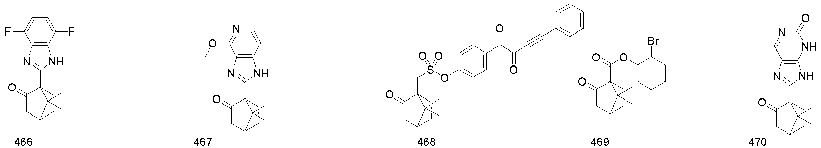

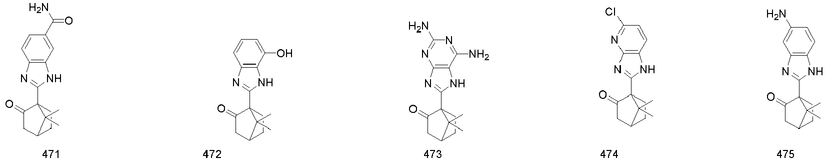

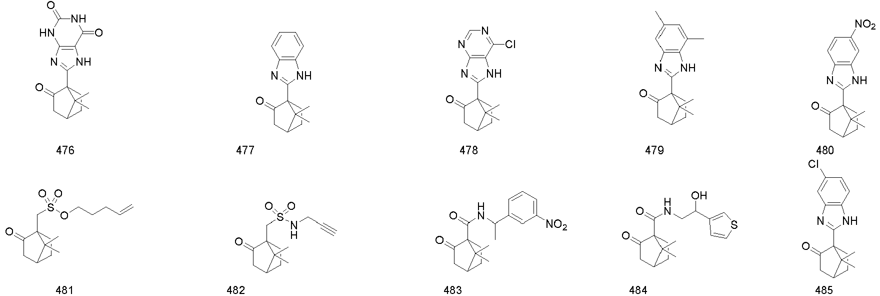

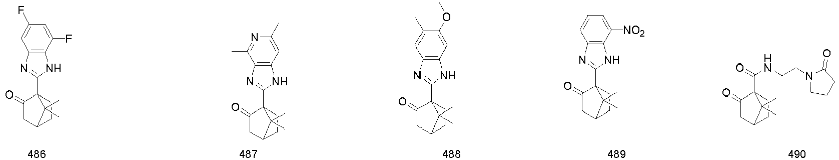

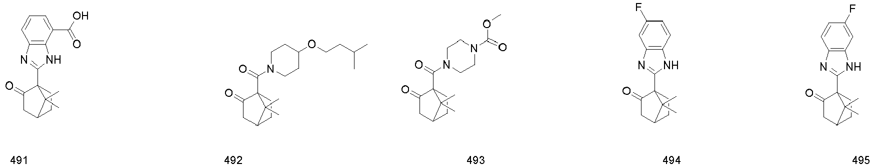

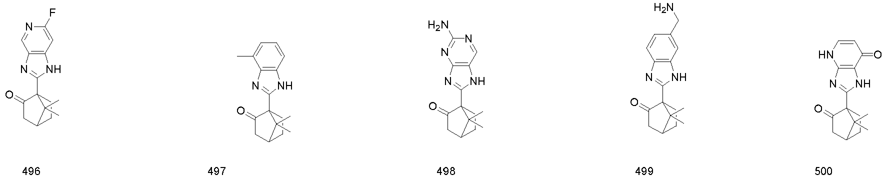

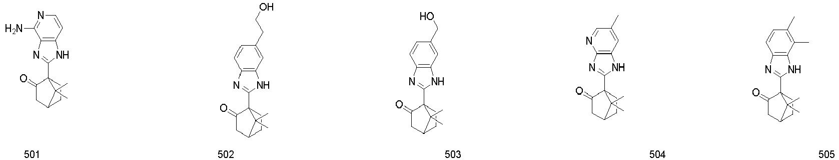

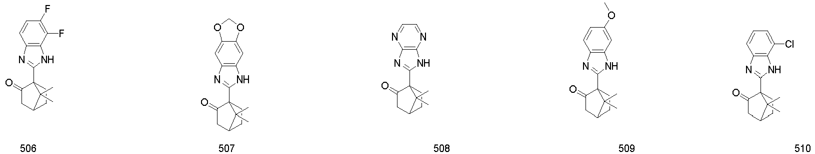

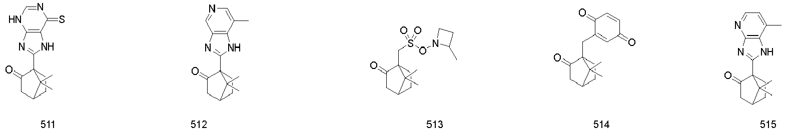

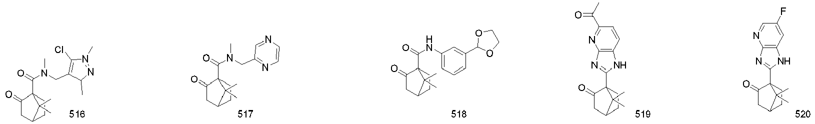

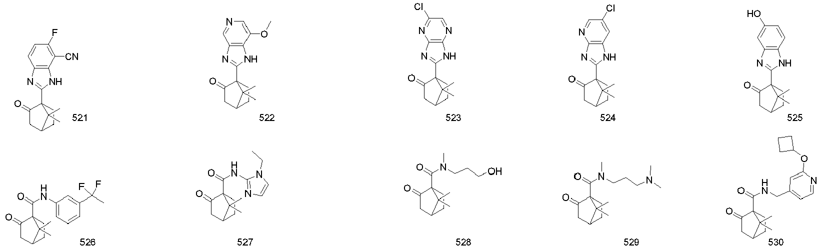

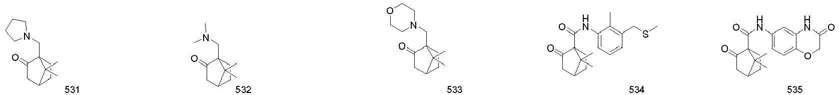

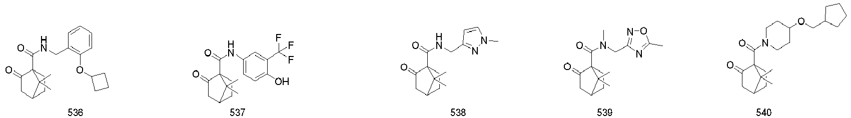

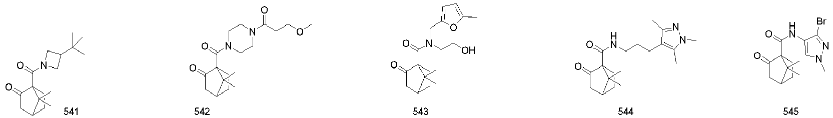

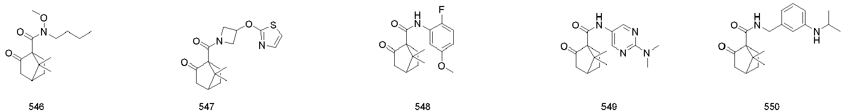

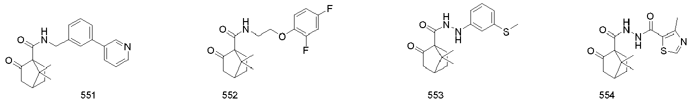

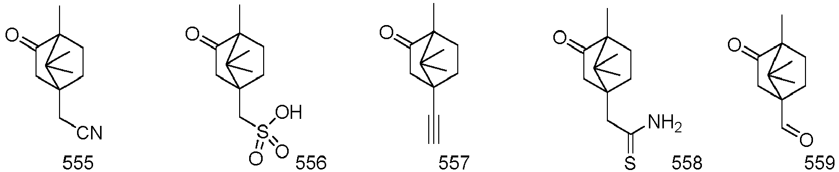

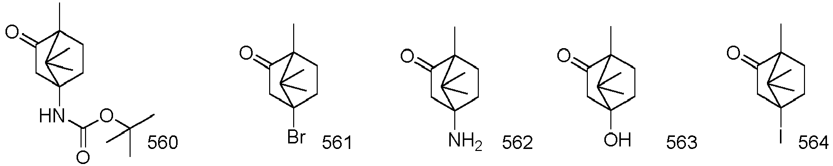

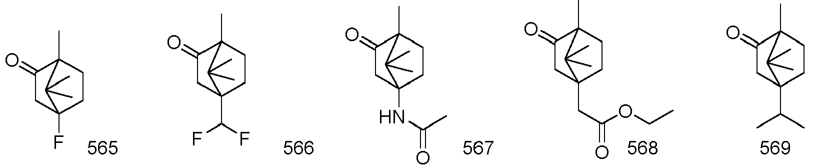

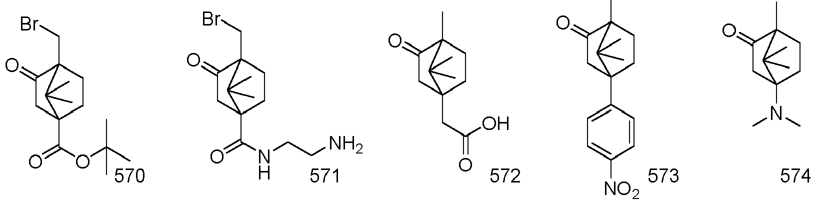

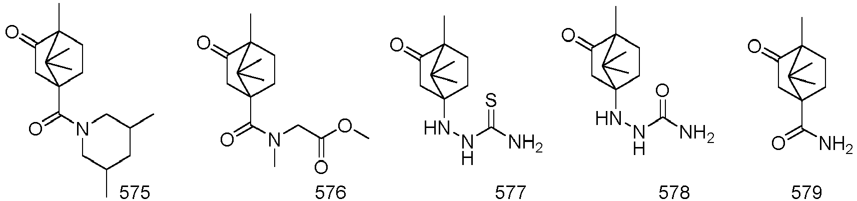

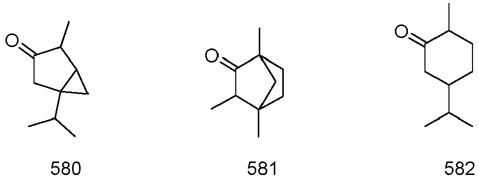

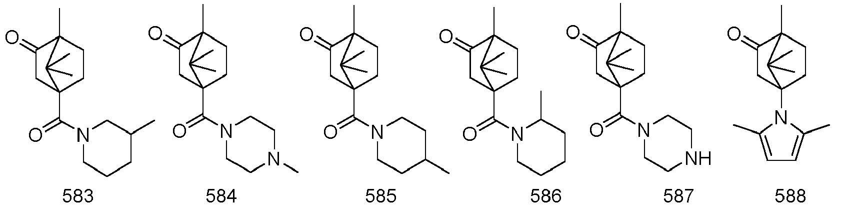

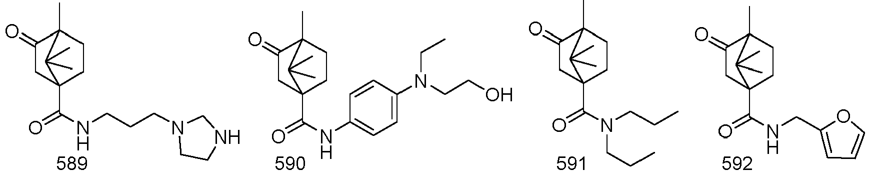

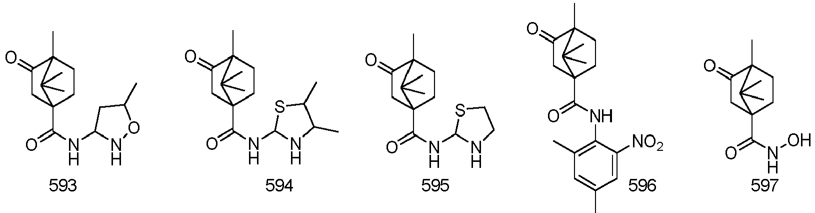

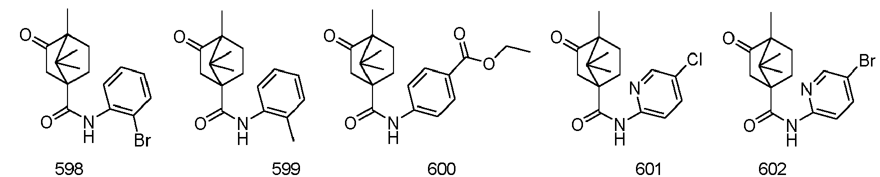

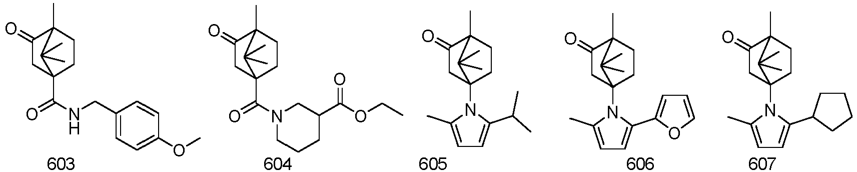

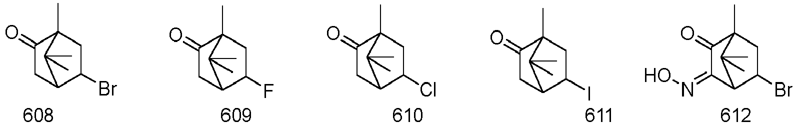

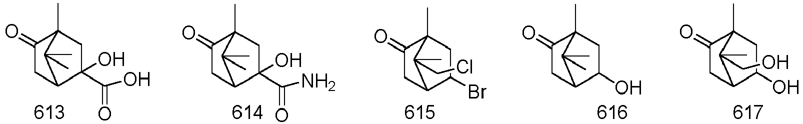

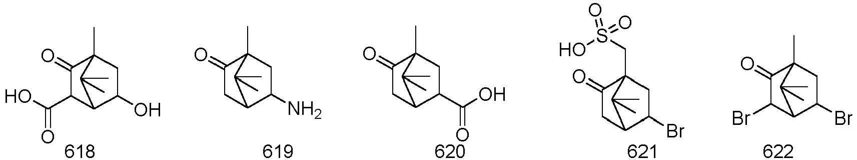

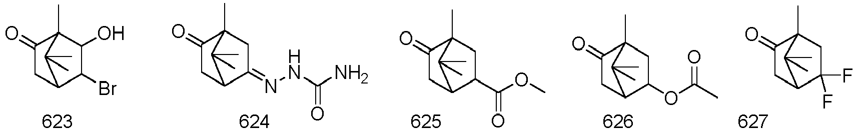

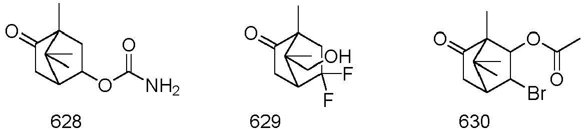

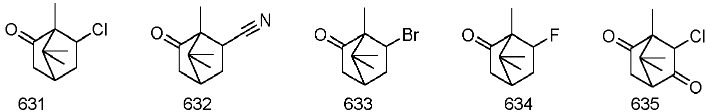

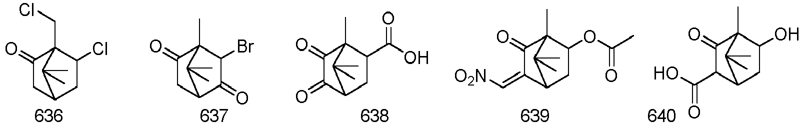

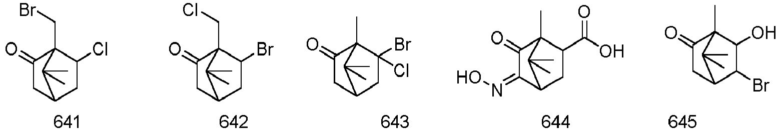

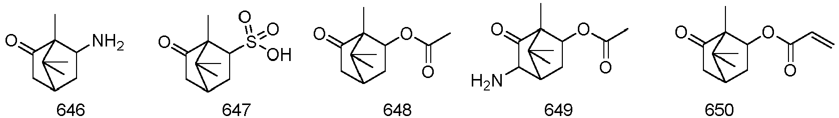

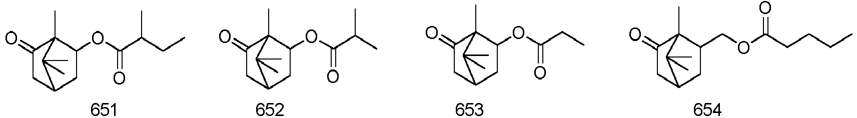

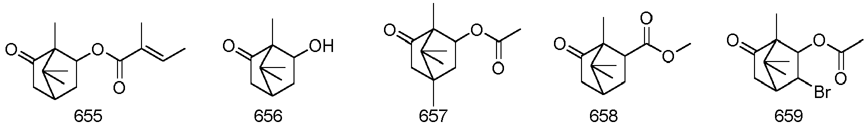

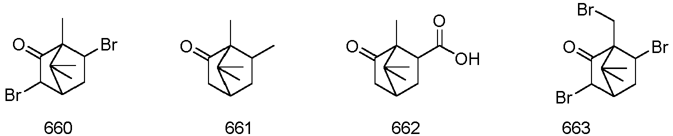

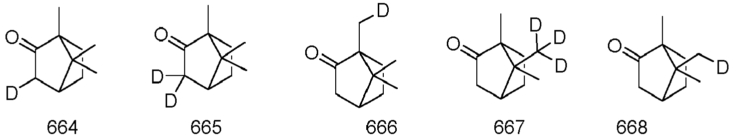

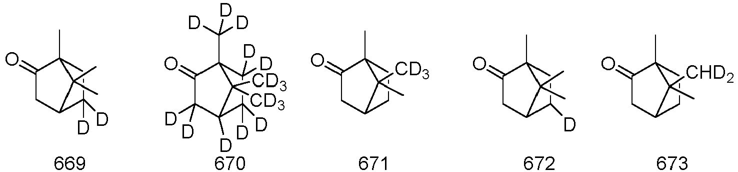

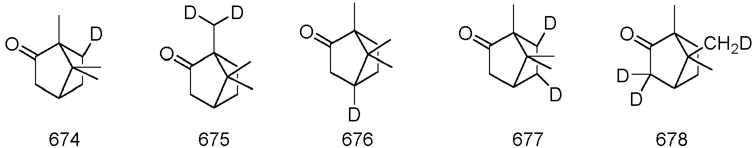

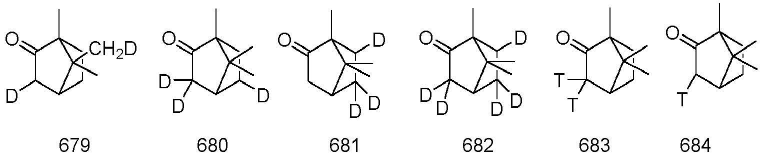

6. The use according to claim 5, **characterized in that** said compound is selected from the group consisting of:

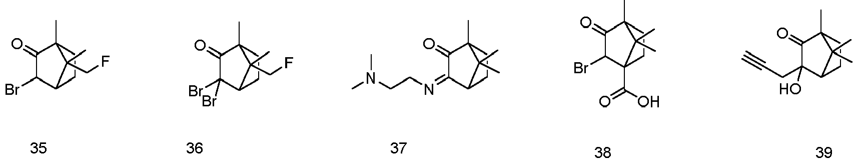

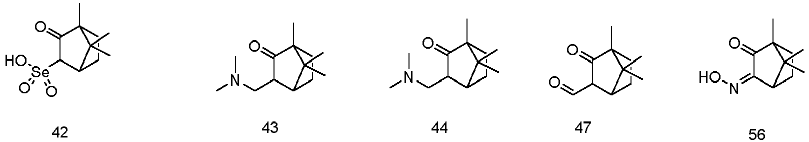

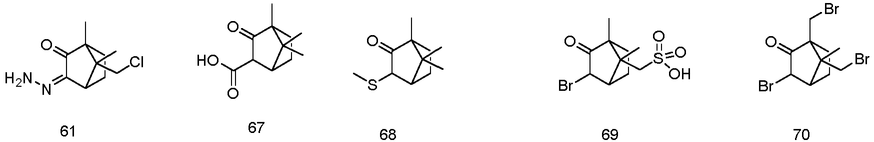

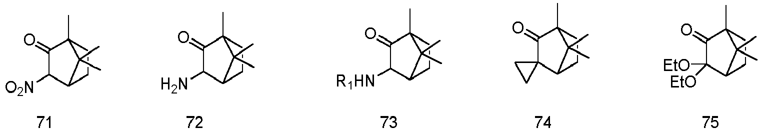

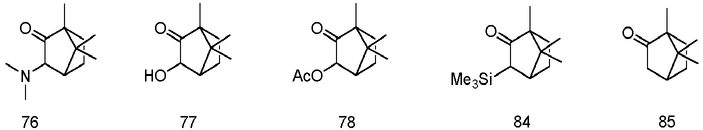

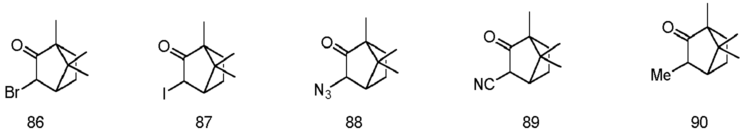

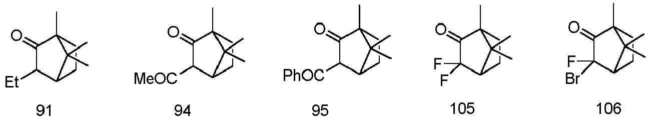

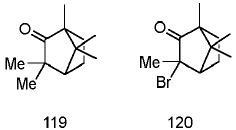

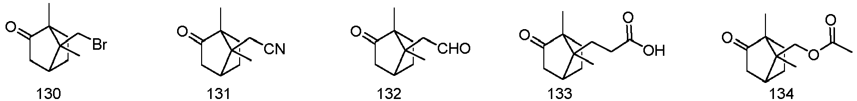

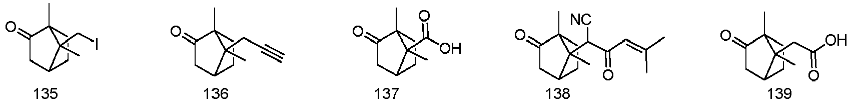

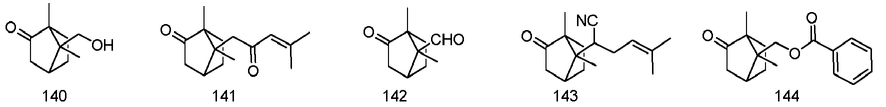

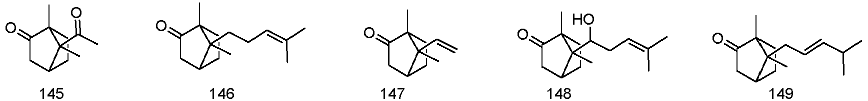

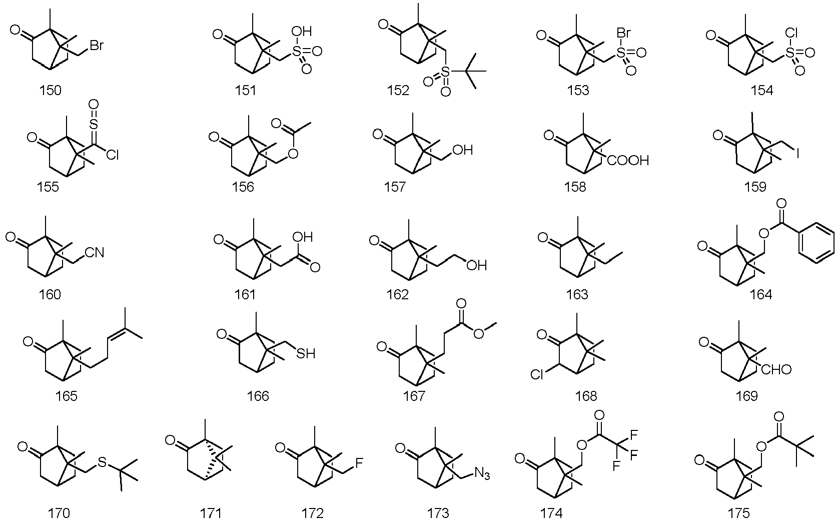

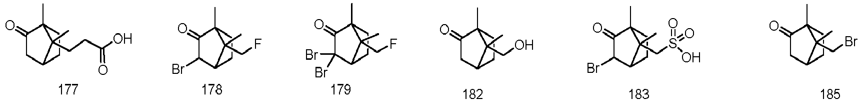

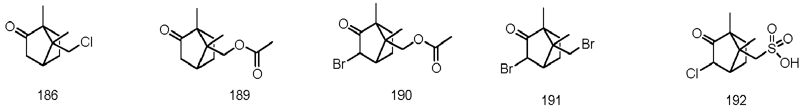

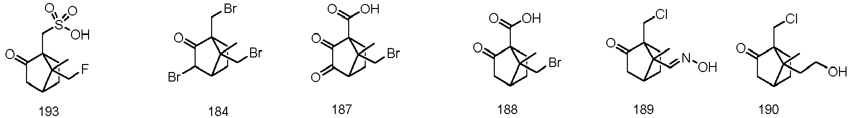

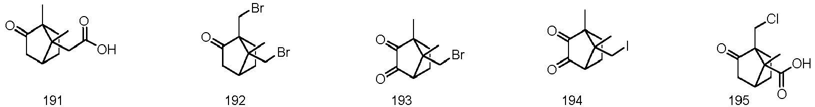

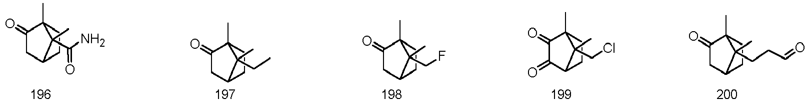

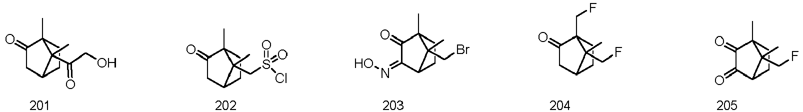

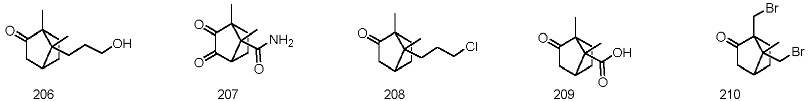

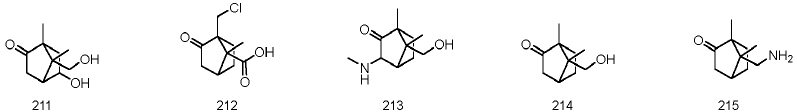

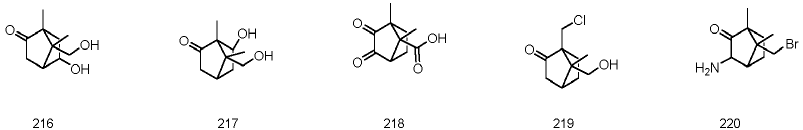

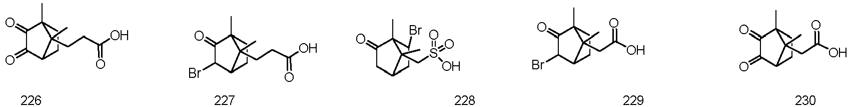

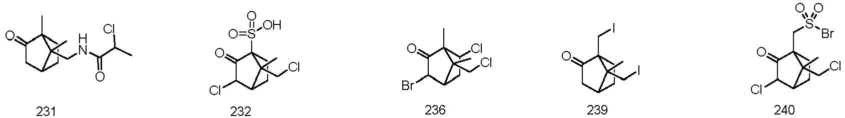

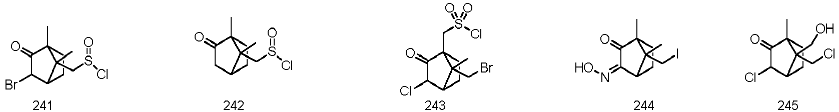

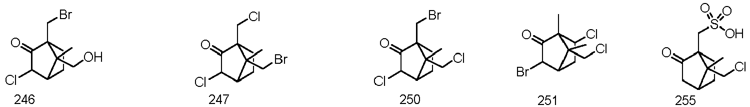

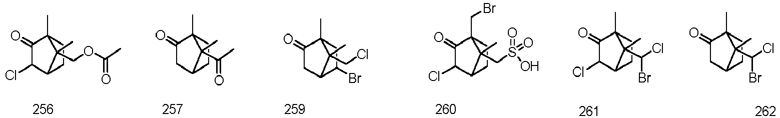

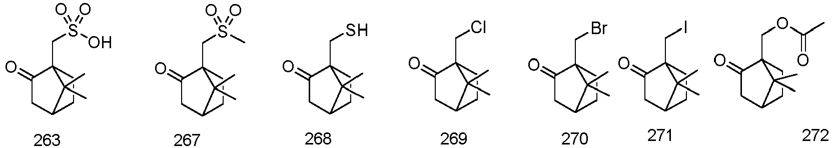

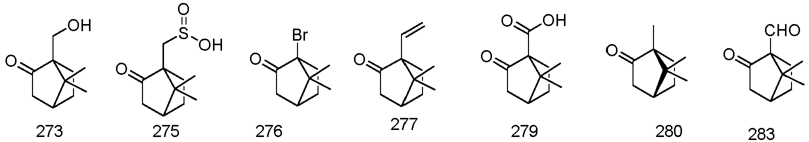

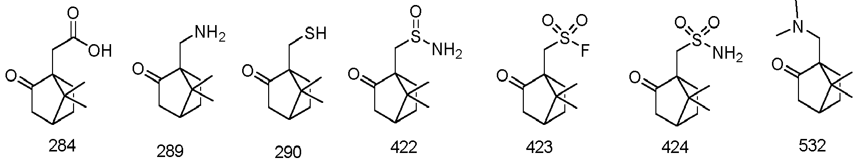

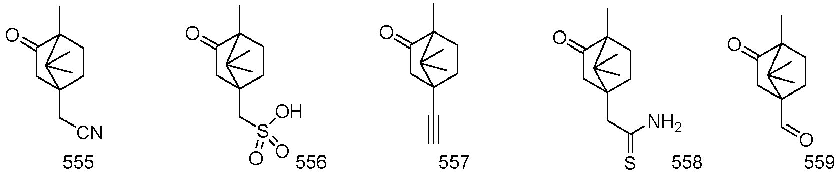

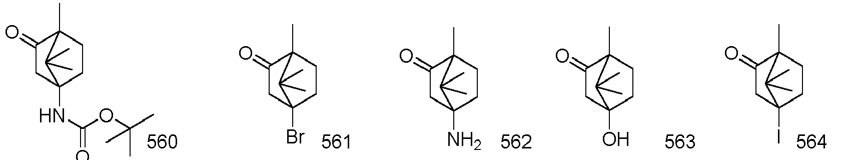

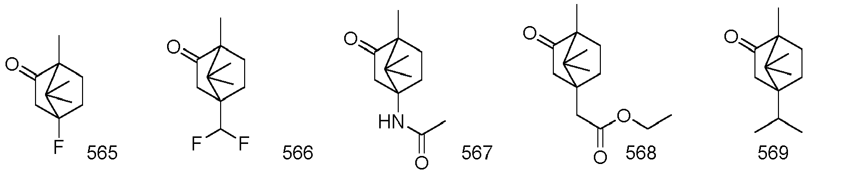

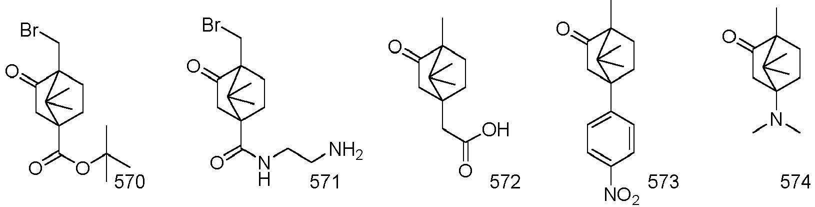

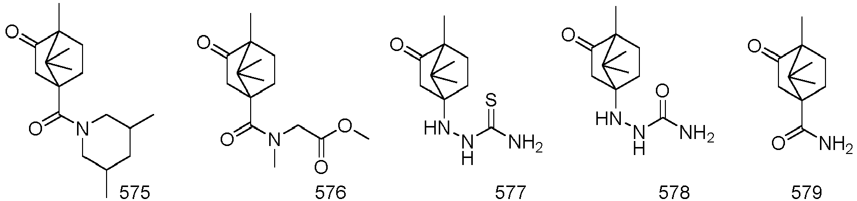

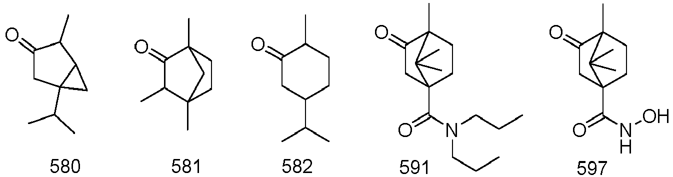

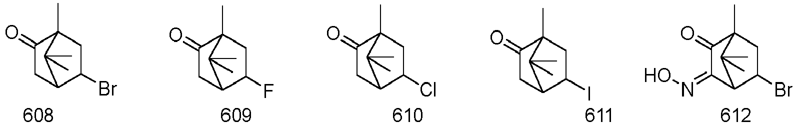

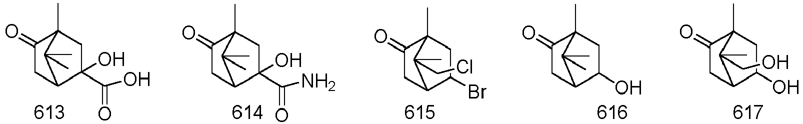

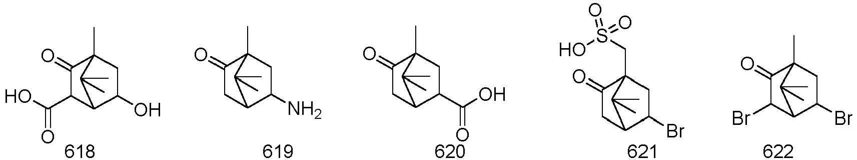

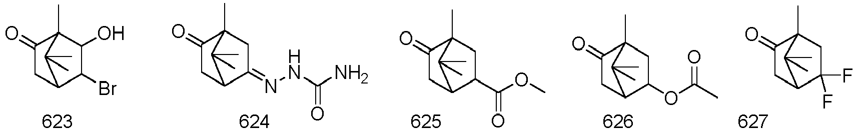

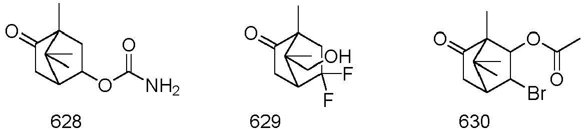

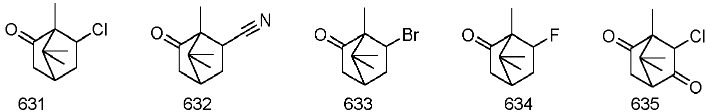

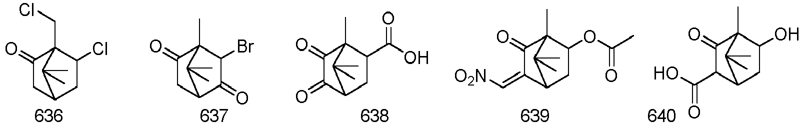

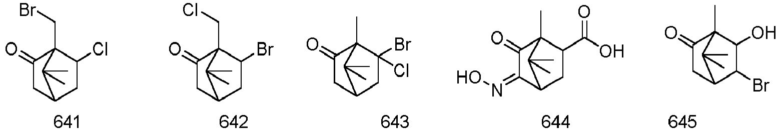

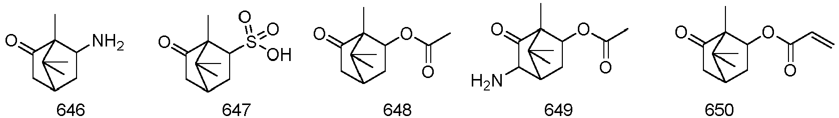

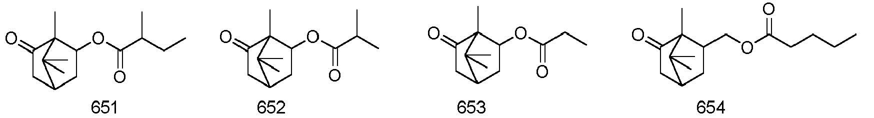

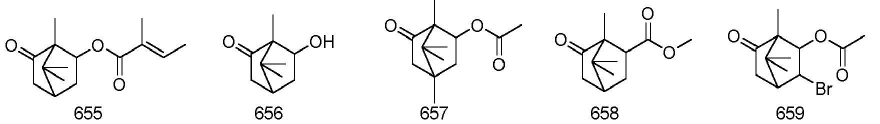

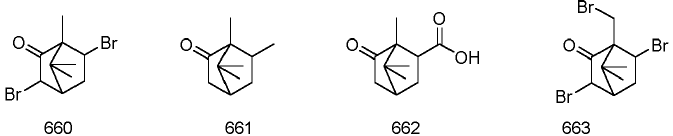

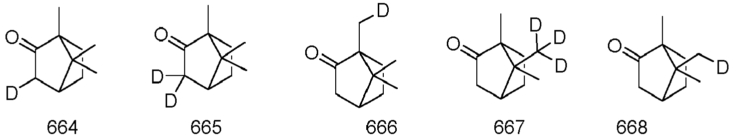

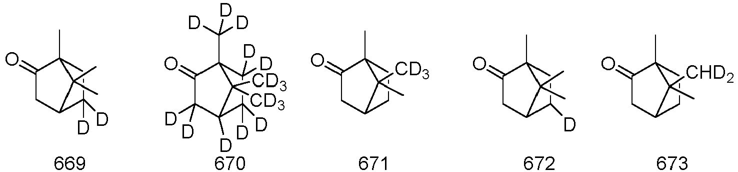

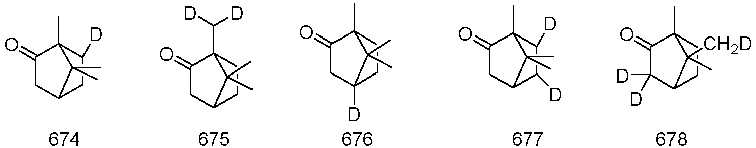

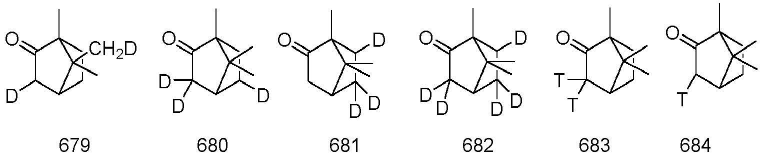

7. The use according to claim 6, **characterized in that** said compound is selected from the group consisting of:

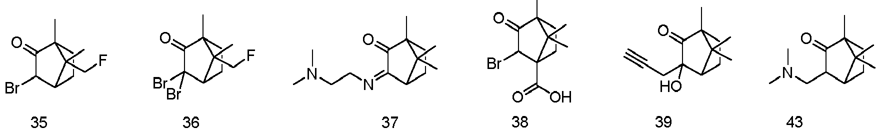

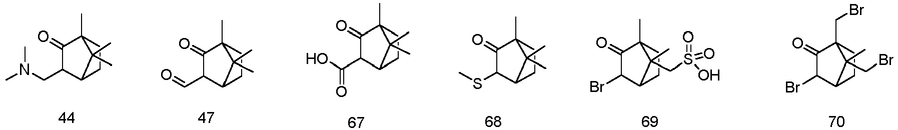

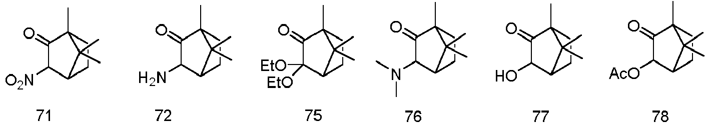

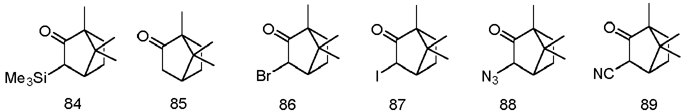

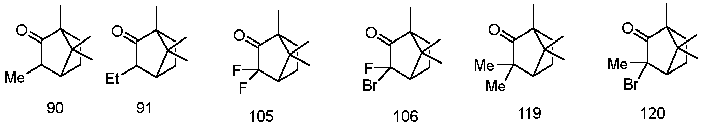

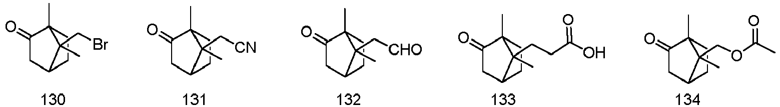

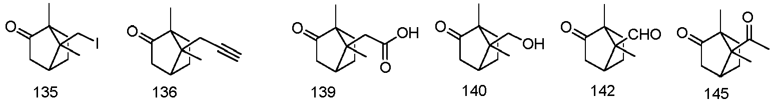

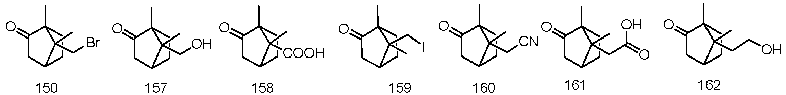

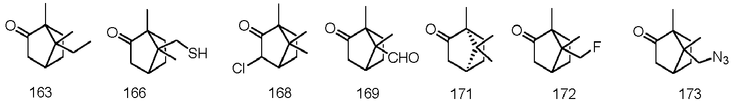

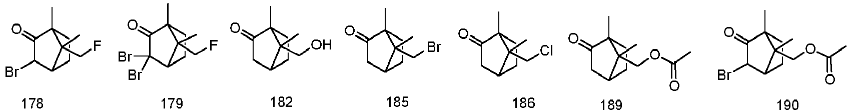

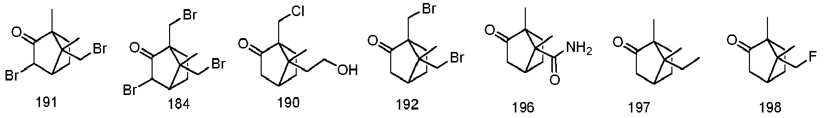

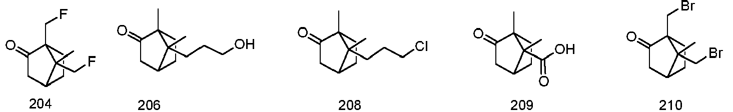

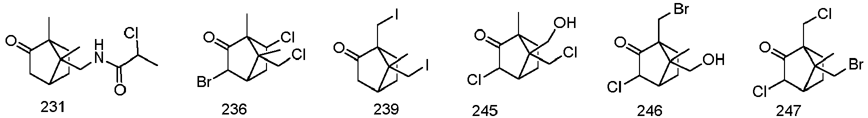

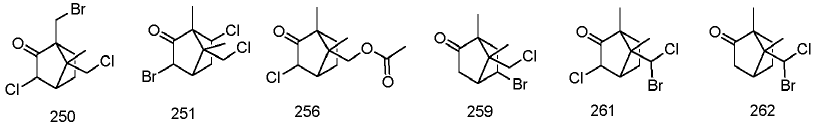

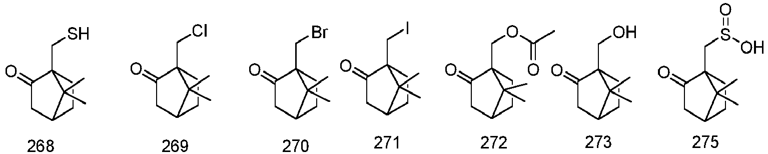

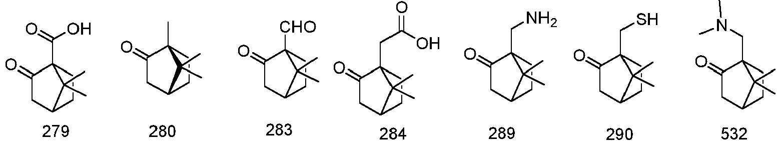

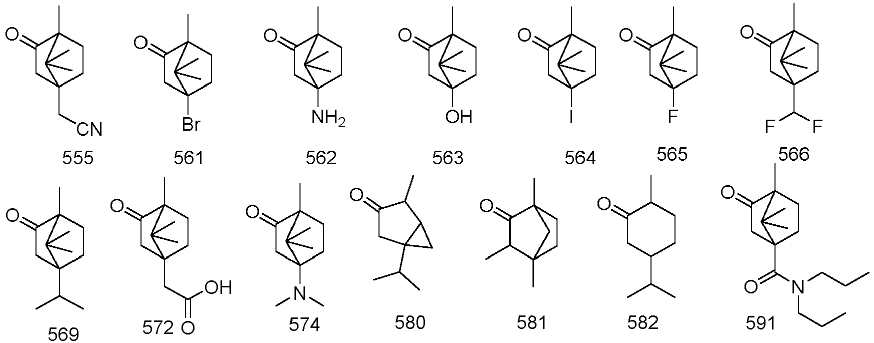

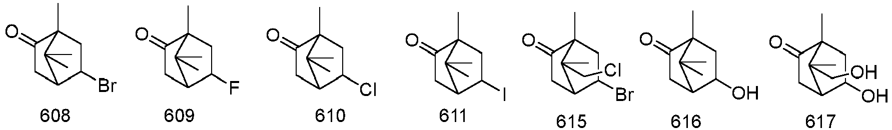

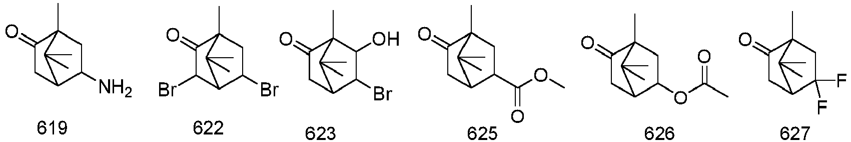

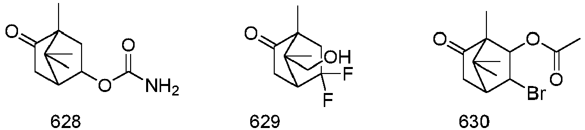

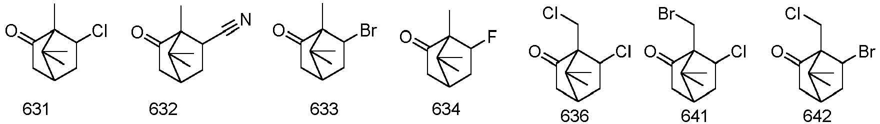

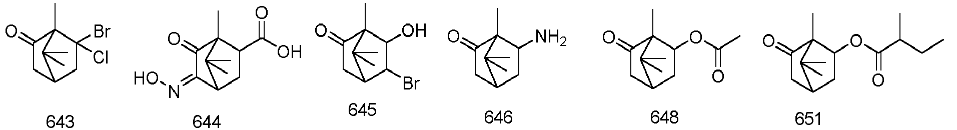

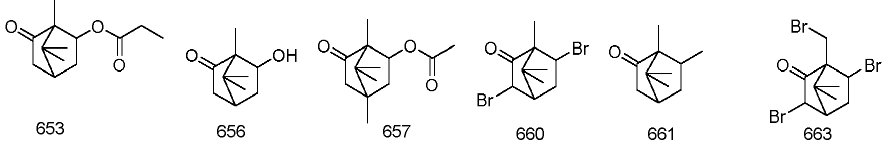

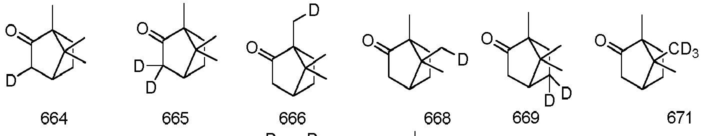

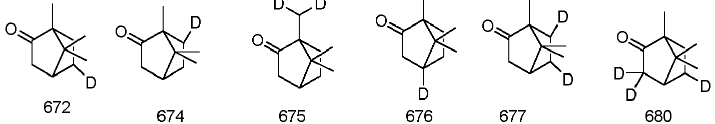

8. The use according to any one of claims 1 to 7, **characterized in that** the anesthetic and/or sedative antagonist is an antagonist of an anesthetic and/or sedative.

9. The use according to claim 8, **characterized in that** the anesthetic and/or sedative antagonist can reverse the anesthetic and/or sedative state induced or maintained by anesthetics and/or sedative drugs.

10. The use according to claim 8, **characterized in that** the anesthetic and/or sedative antagonist can reduce the duration of anesthesia and/or sedation.

11. The use according to claim 8, **characterized in that** the anesthetics include a GABA_{A} receptor agonist.

12. The use according to claim 11, **characterized in that** the anesthetic and/or sedative antagonist is a high affinity ligand of GABA_{A} receptor that can act as a competitive antagonist against GABA_{A} receptor agonists.

13. The use according to claim 8, **characterized in that** the anesthetic is a general anesthetic.

14. The use according to claim 13, **characterized in that** the general anesthesic include benzodiazepine drugs, substituted phenol drugs, imidazole drugs, GABA-like drugs, and phenylcyclohexylamine drugs.

15. The use according to claim 14, **characterized in that** the benzodiazepine drugs include diazepam, midazolam, lorazepam, and remimazolam;
the substituted phenol drugs include propofol and ciprofol;
the imidazole drugs include etomidate or its derivatives;
the GABA-like drugs include γ-aminobutyric acid and γ-hydroxybutyric acid;
the phenylcyclohexylamine drugs include ketamine and fluoketamine.

16. The use according to any one of claims 1 to 15, **characterized in that** the anesthetic and/or sedative antagonist is a preparation prepared with the compounds, or stereoisomers thereof, or deuterated derivatives thereof, or metabolites thereof, or prodrugs thereof, or salts thereof, or solvates thereof, as the active ingredient, in combination with pharmaceutically acceptable excipients.

17. The use according to claim 16, **characterized in that** the preparation is tablet, capsule, oral liquid, granule, pill, powder, injection or powder injection.
